(19)

Europäisches Patentamt

European Patent Office

Office européen des brevets

(11) **EP 4 357 462 A1**

(12) **EUROPEAN PATENT APPLICATION**

(43) Date of publication:
24.04.2024 Bulletin 2024/17

(21) Application number: 22202119.8

(22) Date of filing: 18.10.2022

(51) International Patent Classification (IPC):
**C12Q 1/6886** (2018.01)

(52) Cooperative Patent Classification (CPC):
**C12Q 1/6886;** C12Q 2600/106; C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventor: **HOFFMANN, Ralf Dieter**
**Eindhoven (NL)**

(74) Representative: **Philips Intellectual Property & Standards**
**High Tech Campus 52**
**5656 AG Eindhoven (NL)**

<u>Remarks:</u>
The complete document including Reference Table(s) and the Sequence Listing(s) can be downloaded from the EPO website

(54) **PREDICTION OF AN OUTCOME OF ANDROGEN DEPRIVATION THERAPY IN A PROSTATE CANCER SUBJECT**

(57) The invention relates to a method of predicting a response of a prostate cancer subject to androgen deprivation therapy. The method is based on predicting an outcome based on the expression levels of genes identified herein. The invention provides a method to establish whether androgen deprivation therapy is useful for a subject and thus should be administered or not. Further provided are the use of a kit for determining expression levels for predicting a response of a prostate cancer subject to androgen deprivation therapy.

Fig. 3

EP 4 357 462 A1

**Description**

FIELD OF THE INVENTION

The invention relates to a method of predicting a response of a prostate cancer subject to androgen deprivation therapy, and to a computer program product for predicting a response of a prostate cancer subject to radiotherapy. Moreover, the invention relates to a diagnostic kit, to a use of the kit, to a use of the kit in a method of predicting a response of a prostate cancer subject to androgen deprivation therapy, to a use of a gene expression profile for each of one or more PDE4D7 correlated genes in a method of predicting a response of a prostate cancer subject to radiotherapy, and to a corresponding computer program product.

INTRODUCTION

Cancer is a class of diseases in which a group of cells displays uncontrolled growth, invasion and sometimes metastasis. These three malignant properties of cancers differentiate them from benign tumours, which are self-limited and do not invade or metastasize.

Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol.68, No.6, pages 394-424, 2018). In the US, about 90% of the new cases concern localized cancer, meaning that metastases have not yet been formed (see ACS (American Cancer Society), "Cancer Facts & Figures 2010", 2010). For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid). After treatment, prostate cancer antigen (PSA) levels in the blood are measured for disease monitoring. An increase of the blood PSA level provides a biochemical surrogate measure for cancer recurrence or progression.

Androgen deprivation therapy (ADT), also called androgen suppression therapy, is an antihormone therapy whose main use is in treating prostate cancer. Prostate cancer cells usually require androgen hormones, such as testosterone, to grow. ADT reduces the levels of androgen hormones, with drugs or surgery, to prevent the prostate cancer cells from growing. Neoadjuvant androgen deprivation therapy (NADT) is systemic therapy administered after the diagnosis of prostate cancer but before locoregional therapy such as radical prostatectomy (RP) or radiation. With NADT prior to RP, the intent is to eradicate malignant androgen-dependent cells, in the hope that sufficient tumor regression will permit complete resection of residual prostate cancer, improving pathologic outcome and survival. The role of preoperative androgen deprivation remains controversial, however.

Patients diagnosed with high risk localized prostate cancer have variable outcomes following surgery. Trials of intense NADT have shown lower rates of recurrence among patients with minimal residual disease after treatment. The molecular features that distinguish exceptional responders from poor responders are not known.

Therefore there is an ongoing need for new and improved methods for predicting androgen deprivation therapy response. This unmet need is met by the method and uses as defined in the appended claims.

SUMMARY OF THE INVENTION

In a first aspect the invention relates to a method of predicting a response of a prostate cancer subject to androgen deprivation therapy, comprising:

- determining or receiving the result of a determination of a gene expression profile comprising: the gene expression level of PDE4D7, and/or
- three or more gene expression levels, wherein the three or more gene expression levels are selected from:

  - PDE4D7 correlated genes selected from the group consisting of: ABCC5, CUX2, KIAA1549, PDE4D, RAP1GAP2, SLC39A11, TDRD1, and VWA2,
  said gene expression profile being determined in a biological sample obtained from the subject,

- determining the prediction of the outcome based on the gene expression profile,
- wherein said prediction is a favourable or a non-favourable response to androgen deprivation therapy.

In a second aspect, the invention relates to the use of a diagnostic kit, the kit comprising:

- at least one of: a polymerase chain reaction primer or probes, for determining a gene expression profile in a biological sample obtained from a prostate cancer subject and/or in a sample, the gene expression profile comprising:

  the gene expression level of PDE4D7, and/or
  three or more expression levels, wherein the three or more gene expression levels are selected from:

- PDE4D7 correlated genes selected from the group consisting of: ABCC5, CUX2, KIAA1549, PDE4D, RAP1GAP2, SLC39A11, TDRD1, and VWA2,
  the use comprising predicting a response of a prostate cancer subject to androgen deprivation therapy, wherein the outcome is a favourable or non-favourable response to androgen deprivation therapy.

BRIEF DESCRIPTION OF THE DRAWINGS

Fig 1. Depicts the experimental setup for testing the prostate cancer gene signature on a first cohort of patients.
Fig 2. Depicts the experimental setup for testing the prostate cancer gene signature on a second cohort of patients.
Fig. 3. 35 patients with intermediate/high-risk prostate cancer; all patients treated for neo-adjuvantly with anti-androgens (6 months ADT + Enzalutamide). The figure depicts ROC curves. A testing cohort of 35 patients was used to validate the model. The clinical endpoint used was overall death. The plotted models are the PCAI model and reference model based on a selection of genes that are regulated by the androgen receptor. Area under curve is indicated in the inset.
Fig. 4. 24 patients (43 samples) with localized, high-risk prostate cancer; all patients treated for 6 months neo-adjuvantly with anti-androgens (6 months enzalutamide, lupron, abiraterone, apalutamide). The figure depicts ROC curves. A testing cohort of 35 patients was used to validate the model. The clinical endpoint used was overall death. The plotted models are the PCAI model and reference model based on a selection of genes that are regulated by the androgen receptor. Area under curve is indicated in the inset.

DEFINITIONS

As used herein the indefinite term "a" or "an" does not exclude a plurality.
The term "biological sample" or "sample obtained from a subject" refers to any biological material obtained via suitable methods known to the person skilled in the art from a subject, e.g. a prostate cancer subject.
As used herein, the term "and/or" indicates that one or more of the stated cases may occur, alone or in combination with at least one of the stated cases, up to with all of the stated cases.
As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.
The term "prostate cancer" refers to a cancer of the prostate tissue, which occurs when cells in the prostate mutate and begin to grow out of control.
The term "prostate cancer specific death or disease specific death" refers to death of a patient from a prostate cancer.
The term "clinical recurrence" refers to the presence of clinical signs indicating the presence of tumour cells as measured, for example using in vivo imaging.
As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of' and "consisting of".
When used herein, the term "immune defense response genes" is interchangeably used with "IDR genes" or "immune defense genes" refers to one or more of the genes selected from AIM2, APOBEC3A, CIAO1, DDX58, DHX9, IFI16, IFIH1, IFIT1, IFIT3, LRRFIP1, MYD88, OAS1, TLR8, and ZBP1.
The term "metastases" refers to the presence of metastatic disease in organs other than a prostate tissue.
When used herein, the term "PDE4D7 correlated genes" is interchangeably used with "PDE4D7 genes" refers to one or more of the genes selected from: ABCC5, CUX2, KIAA1549, PDE4D, RAP1GAP2, SLC39A11, TDRD1, and VWA2.
When used herein, the term "T-cell receptor signalling genes" is interchangeably used with "TCR signalling genes" or "TCR genes" refers to one or more of the genes selected from: CD2, CD247, CD28, CD3E, CD3G, CD4, CSK, EZR, FYN, LAT, LCK, PAG1, PDE4D, PRKACA, PRKACB, PTPRC, and ZAP70.

When used herein the term "outcome" or "prediction of a outcome" refers to a response of a prostate cancer subject to androgen deprivation therapy.

When used herein "PCAI Immunoscore" refers to a model based on the PDE4D7 correlated genes. Therefore the PCAI Immunoscore provides a model based on the expression levels of each of ABCC5, CUX2, KIAA1549, PDE4D, RAP1GAP2, SLC39A11, TDRD1, and VWA2, wherein each expression level is assigned a weight in the model. The used model is described in WO 2022/043120 , which is hereby incorporated by reference in its entirety.

When used herein the term "gene expression profile" refers to a signature of one or more expression levels, particularly the PDE4D isoform 7 expression level and/or three or more of the PDE4D7 correlated genes as defined herein. Therefore, the gene expression profile may comprise solely the expression level for PDE4D7 (that is isoform 7 of the phosphodiesterase 4D gene). The gene expression profile may for example also comprise the PDE4D7 expression level and one or more additional expression levels, such as but not limited to one or more expression levels selected from the PDE4D7 correlated genes, one or more expression levels selected from the immune defense response genes and/or one or more expression levels of the T-Cell Receptor Signalling genes as defined herein.

## DETAILED DESCRIPTION OF THE INVENTION

### Immune system in cancer

In recent years, the importance of the immune system in cancer inhibition as well as in cancer initiation, promotion and metastasis has become very evident (Mantovani et al. Nature. 454(7203):436-44 (2008); Giraldo et al. Br J Cancer. 120(1):45-53 (2019)). The immune cells and the molecules they secrete form a crucial part of the tumour microenvironment and most immune cells can infiltrate the tumour tissue. The immune system and the tumour affect and shape one another. Thus, antitumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (Giraldo Br J Cancer. 120(1):45-53 (2019)).

In summary, the status of the immune system and of the immune microenvironment have an impact on therapy effectiveness.

The inventors have identified gene signatures, and combination of these signatures with clinical parameters, of which the resulting models show a significant relation to mortality and therefore are expected to improve the prediction of the effectiveness of treatments.

### PDE4D7 correlated genes

Phosphodiesterases (PDEs) provide the sole means for the degradation of the second messenger 3'-5'-cyclic AMP. As such they are poised to provide a key regulatory role. Thus, aberrant changes in their expression, activity and intracellular location may all contribute to the underlying molecular pathology of particular disease states. Indeed, it has recently been shown that mutations in PDE genes are enriched in prostate cancer patients leading to elevated cAMP signalling and a potential predisposition to prostate cancer. However, varied expression profiles in different cell types coupled with complex arrays of isoform variants within each PDE family makes understanding the links between aberrant changes in PDE expression and functionality during disease progression challenging. Several studies have endeavoured to describe the complement of PDEs in prostate, all of which identified significant levels of PDE4 expression alongside other PDEs, leading to the development of a PDE4D7 biomarker (see Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018). Since the PDE4D7 biomarker has been proven to be a good predictor, it was assumed that the ability to identify markers that are highly correlated with the PDE47 biomarker might also be helpful in prognosticating the outcome of certain cancer subjects.

Based on the correlation between PDE4D7 expression and pathological features of the disease, the defined aim was to identify prognostic associations between the expression of PDE4D7 in a patient prostate tissue, collected by either biopsy or surgery, and clinically useful information relevant to the outcome of individual patients. Clinically relevant endpoints, or surrogate endpoints that are significantly correlated to the development of metastases, cancer specific or overall mortality have, typically, been evaluated as prognostic cancer biomarkers. The most relevant rational for using a surrogate endpoint relates to situations where either data on established clinical endpoints are not available or when the number of events in the data cohort is too limited for statistical data analysis. For the development of the PDE4D7 prognostic biomarker either BCR (biochemical relapse) progression-free survival or start of post-surgical secondary treatment were evaluated as surrogate endpoints for metastases and prostate cancer death. Using these particular endpoints, a relevant number of events in the selected clinical cohorts (e.g., >30% for BCR) were identified, which is particularly relevant for multivariable data analysis.

In the performed evaluation, standard methods of multivariable analysis such as Cox regression and Kaplan-Meier survival analysis were selected in order to investigate the added and independent value of the continuous and/or the categorical 'PDE4D7 score' compared to established prognostic clinical variables such as PSA and Gleason score (Alves de Inda, 2018). Risk models were built wherein the 'PDE4D7 score' was combined with either pre- or post-surgical clinical predictors of post-surgical progression using logistic regression. The resulting models were subsequently tested on multiple independent patient cohorts in Kaplan-Meier survival and ROC curve analysis in order to predict post-treatment progression free survival (Alves de Inda, 2018).

Using such a strategy, the prognostic value of the PDE4D7 score on a biopsy from retrospectively collected, resected prostate tissue in a consecutively managed patient cohort from a single surgery center in a post-surgical setting (Alves de Inda, 2018) was tested. The patient population comprised some 500 individuals where longitudinal follow-up, of both pathology and biological outcomes, was undertaken. These clinical data were available for all patients and collected during a follow-up of a median 120 months after treatment. The 'PDE4D7 score' was determined as described above and then tested in both uni- and multivariable analyses using the available post-surgical co-variates (i.e. pathology Gleason score, pT stage, surgical margin status, seminal vesicle invasion status, and lymph node invasion status) in order to adjust for the multivariable setting. In this instance, biochemical progression-free survival after primary intervention was set as the evaluated clinical endpoint. The univariable analysis of these clinical samples (Alves de Inda, 2018), showing the inverse association between PDE4D7 expression (in terms of 'PDE4D7 score') and post-surgical biological relapse (HR=0.53 per unit change; 95% CI 0.41-0.67; p<0.0001), robustly confirmed previous data (Boettcher 2015; Boettcher, 2016). In multivariable analysis with such clinical variables, the 'PDE4D7 score' remained as an independent and effective means for predicting clinical outcome (HR=0.56 per unit change; 95% CI 0.43-0.73; p<0.0001). Furthermore, a very similar outcome was obtained when the 'PDE4D7 score' in multivariable analysis (HR=0.54 95% CI 0.42-0.69; p<0.0001) with the validated and clinically-used risk model CAPRA-S was evaluated. The CAPRA-S score, which is based on pre-operative PSA and pathologic parameters determined at the time of surgery, was developed to provide clinicians with information aimed to help predict disease recurrence, including BCR, systemic progression, and PCSM and has been validated in US and other populations.

Interestingly, when assessing the hazard ratio (HR) compared to the continuous 'PDE4D7 score' a linear increase in risk with decreasing 'PDE4D7 score' for score values lying between 2 and 5 was uncovered. However, at PDE4D7 scores less than 2, then the risk of post-surgical progression increases steeply (Alves de Inda, 2018). This is also evident in the Kaplan-Meier survival curves where patients that are grouped within the lowest 'PDE4D7 scores' category exhibit the highest risk of disease recurrence. Using logistic regression analysis, the CAPRA-S score was combined with the continuous 'PDE4D7 score'. Testing this model using ROC curve analysis a 4-6% significant improvement in AUC was noticed when compared to the CAPRA-S alone for both 2- and 5-year predictions of post-treatment progression to BCR. Thus, a combined CAPRA-S & 'PDE4D7 score' Cox regression combination model was evaluated in Kaplan-Meier survival analysis and compared this to the CAPRA-S score categories alone. Undertaking this, it was confirmed that the added value in risk prediction when using a model, the combined 'PDE4D7 & CAPRA-S' score, compared to using the clinical metric of CAPRA-S score alone (Alves de Inda, 2018).

Subsequent to the diagnosis of prostate cancer, an accurate risk assessment needs to be undertaken before stratification to a defined primary treatment. With this in mind, it was tested whether it was possible to translate the prognostic use of the 'PDE4D7 score' in a pre-surgery situation testing tumour tissue obtained from diagnostic needle biopsy samples (van Strijp 2018). In this, needle biopsies were performed on 168 patients, from a single diagnostic clinical centre, who had undergone surgery as a primary treatment. The minimum follow-up period for each patient was 60 months after this intervention. The clinical co-variates used to adjust the 'PDE4D7 score' in the multivariable analysis were age at surgery, pre-operative PSA, PSA density, biopsy Gleason score, percentage of tumour positive biopsy cores, percentage of tumour in the biopsy and clinical cT stage. In this the utility of the 'PDE4D7 score' and the combined 'PDE4D7 & CAPRA' scores compared to the pre-surgical CAPRA score in Cox regression analysis for biochemical relapse (van Strijp 2018) were evaluated.

Evaluating this patient cohort it was found (van Strijp 2018) that the 'PDE4D7 score' was inversely associated with BCR in multivariable analysis when adjusting for clinical variables (HR=0.43; 95% CI 0.29-0.63; p<0.0001) as well as for the clinical CAPRA score (HR=0.53; 95% CI 0.38-0.74; p=0.0001). Kaplan-Meier analysis demonstrated that, as before, in a post-surgical setting, the 'PDE4D7 score' categories were significantly associated with BCR progression free survival (logrank p<0.0001) and secondary treatment free survival (logrank p=0.01). Next a combination logistic regression model, which was developed on the previous cohort, was employed (van Strijp 2018). This consisted of the combined 'CAPRA & PDE4D7' score, demonstrating that patients within the highest combined 'CAPRA & PDE4D7' combined score category have virtually no risk of biochemical progression or transfer to any secondary treatment after surgery. This logistic regression model was also evaluated using ROC curve analysis in order to predict 5-year BCR after surgery. This revealed an increase in AUC of 5% over the CAPRA score alone (AUC=0.82 vs. 0.77, respectively; p=0.004). Decision curve analysis of the combined 'CAPRA & PDE4D7' score model confirmed the superior net benefit of using this combined score, compared to either score alone, across all decision thresholds in order to decide on whether to undertake intervention (e.g. surgery) based on the risk threshold of an individual patient to experience post-surgical disease progression (van Strijp 2018).

The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the treatment response, in order to increase the success rate of these therapies.

The identification and role of the PDE4D7 correlated genes has been described in WO 2022/043120 hereby incorporated by reference in its entirety.

Ideally the expression level of the PDE4D isoform 7 is therefore used in a predictive model, however depending on the method of detection isoform specific expression data may not always be available. In such case there may only be general expression level available covering all PDE4D isoforms. Therefore a PDE4D7 specific gene signature (PDE4D7 correlated genes) may be used which is based on the expression levels of genes which correlate specifically with the PDE4D7 isoform expression level.

**Immune response defense genes**

The integrity and stability of genomic DNA is permanently under stress induced by various cell internal and external factors like exposure to radiation, viral or bacterial infections, but also oxidation and replication stress (see Gasser S. et al. "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g. DNA unnaturally appearing in the cytosolic fraction of the cell in contrast to the nucleus) and damaged DNA (e.g. through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

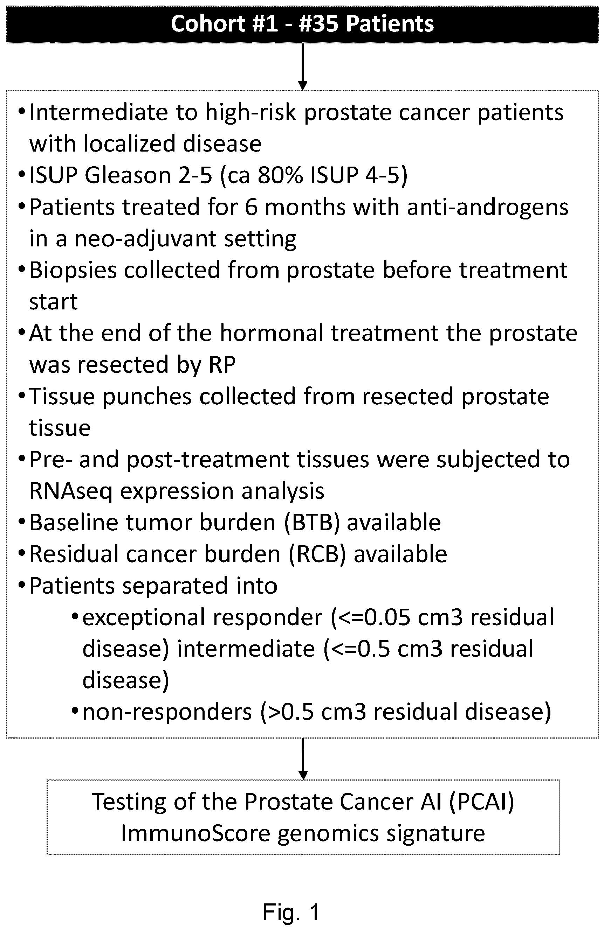

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-lbeta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF- kB and interferon responses (light green) are shown).

The factors and mechanisms responsible for activating the DNA sensor pathways in cancer are currently not well elucidated. It will be important to identify the intratumoural DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

The identification and role of the immune response defense genes has been described in WO 2021/175746 hereby incorporated by reference in its entirety.

**T-Cell receptor signalling genes**

An immune response against pathogens can be elicited at different levels: there are physical barriers, such as the skin, to keep invaders out. If breached, innate immunity comes into play; a first and fast non-specific response. If this is not sufficient, the adaptive immune response is elicited. This is much more specific and needs time to develop when encountering a pathogen for the first time. Lymphocytes are activated by interacting with activated antigen presenting cells from the innate immune system and are also responsible for maintenance of memory for faster responses upon next encounters with the same pathogen.

As lymphocytes are highly specific and effective when activated, they are subject to negative selection for their ability to recognize self, a process known as central tolerance. As not all self-antigens are expressed at selection sites, peripheral tolerance mechanisms evolved as well, such as ligation of the TCR in absence of co-stimulation, expression of inhibitory co-receptors, and suppression by Tregs. A disturbed balance between activation and suppression may lead to autoimmune disorders, or immune deficiencies and cancer, respectively.

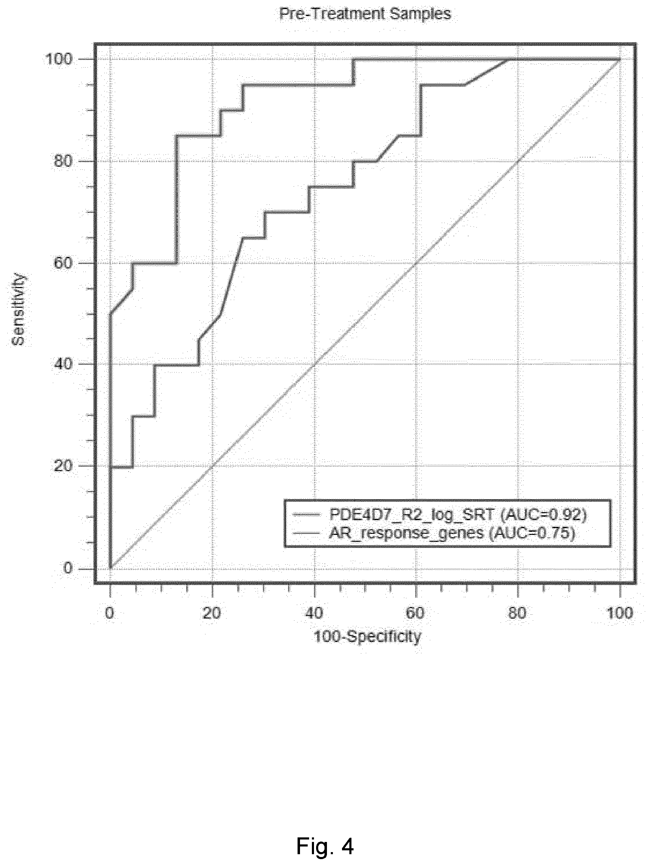

T-cell activation can have different functional consequences, depending on the location the type of T-cell involved. CD8+ T-cells differentiate into cytotoxic effector cells, whereas CD4+ T-cells can differentiate into Th1 (IFN$\gamma$ secretion and promotion of cell mediated immunity) or Th2 (IL4/5/13 secretion and promotion of B cell and humoral immunity). Differentiation towards other, more recently identified T-cell subsets is also possible, for example the Tregs, which have a suppressive effect on immune activation (see Mosenden R. and Tasken K., "Cyclic AMP-mediated immune regulation - Overview of mechanisms of action in T-cells", Cell Signal, Vol. 23, No. 6, pages 1009-1016 (2011), in particular, Fig. 4, which T-cell activation and its modulation by PKA, and Tasken K. and Ruppelt A., "Negative regulation of T-cell receptor activation by the cAMP-PKA-Csk signalling pathway in T-cell lipid rafts", Front Biosci, Vol. 11, pages 2929-2939 (2006)).

T cell activation can take place in naive and differentiated T cells. On a molecular level, the events following the ligation of the TCR with cognate antigen, and crosstalk with signalling induced by co-stimulatory and co-inhibitory receptors determine whether the T cell will become activated or if it will become anergic. Just triggering the TCR itself is not enough, this leads to T cell anergy. The B7:CD28 family of co-stimulatory molecules plays a central role in controlling the activation status of T cells upon antigenic stimulation (Torheim E.A., "Immunity Leashed - Mechanisms of Regulation in the Human Immune System", Thesis for the degree of Philosophiae Doctor (PhD), The Biotechnology Centre of Ola, University of Oslo, Norway, 2009).

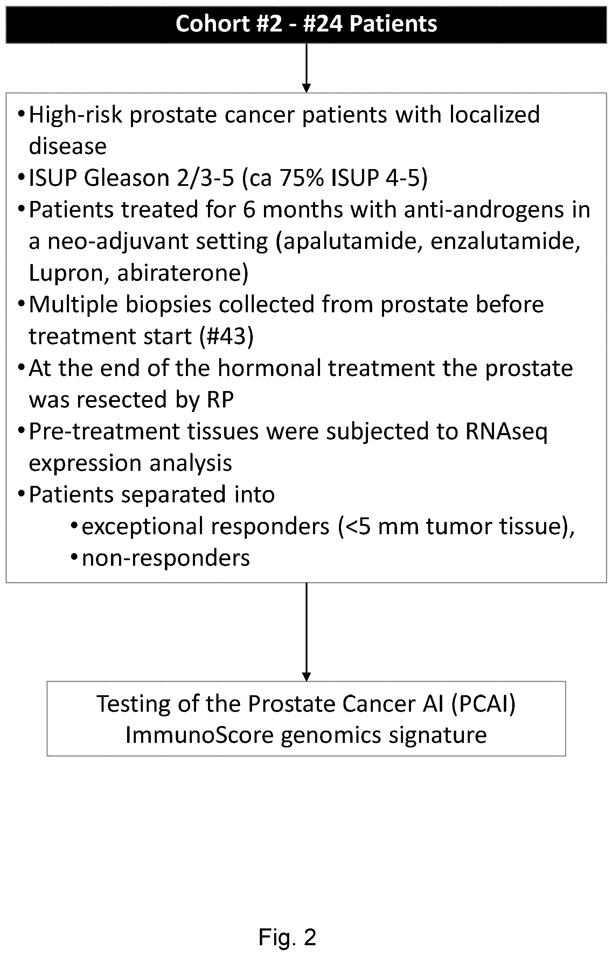

Activation occurs upon interaction of the TCR on the surface of the T cell with MHC-peptide complexes on APCs or target cells (Figure 2). An immunological synapse is formed, lipid rafts in the T cell membrane coalesce and Lck and Fyn are activated. These molecules phosphorylate ITAMs in the CD3 subunits of the TCR, which facilitates recruitment of Zap-70 in proximity with Lck. Lck phosphorylates and activates Zap-70, which in turn then phosphorylates LAT, SLP76, and PLC$\gamma$1. LAT is a docking site for other signalling molecules and is essential for downstream TCR signalling. Grb2, Gads, PI3K and NCK are recruited to LAT, propagating signalling involving activation of RAS, PKC, mobilization of Ca2+, calcineurin and polymerization of the actin cytoskeleton (Tasken et al. Front in Bioscience 11:2929-2939 (2006)). This eventually leads to activation of transcription factors of the NFkB, NFAT, API, and ATF families, resulting in transcription of genes for immune activation (Mosenden et al. Cell Signalling 23:1009-1016 (2011); Tasken et al. Front in Bioscience 11:2929-2939 (2006)).

Both PKA and PDE4 regulated signalling intersect with TCR induced T-cell activation to fine-tune its regulation, with opposing effects (see Abrahamsen H. et al., "TCR- and CD28-mediated recruitment of phosphodiesterase 4 to lipid rafts potentiates TCR signaling", J Immunol, Vol. 173, pages 4847-4848 (2004), in particular, Fig. 6, which shows opposing effects of PKA and PDE4 on TCR activation). The molecule that connects these effectors is cyclic AMP (cAMP), an intracellular second messenger of extracellular ligand action. In T-cells, it mediates effects of prostaglandins, adenosine, histamine, beta-adrenergic agonists, neuropeptide hormones and beta-endorphin. Binding of these extracellular molecules to GPCRs leads to their conformational change, release of stimulatory subunits and subsequent activation of adenylate cyclases (AC), which hydrolyse ATP to cAMP (see Fig. 6 of Abrahamsen H. et al., 2004, ibid). Although not the only one, PKA is the principal effector of cAMP signalling (see Mosenden R. and Tasken K., 2011, ibid, and Tasken K. and Ruppelt A., 2006, ibid). At a functional level, increased levels of cAMP lead to reduced IFN$\gamma$ and IL-2 production in T-cells (see Abrahamsen H. et al., 2004, ibid). Aside from interfering with TCR activation, PKA has many more effectors (see Fig. 15 of Torheim E.A., 2009, ibid).

In naive T-cells, hyperphosphorylated PAG targets Csk to lipid rafts. Via the Ezrin-EBP50-PAG scaffold complex PKA is targeted to Csk. Through specific phosphorylation by PKA, Csk can negatively regulate Lck and Fyn to dampen their activity and downregulate T-cell activation (see Fig. 6 of Abrahamsen H. et al., 2004, ibid). Upon TCR activation, PAG is dephosphorylated and Csk is released from the rafts. Dissociation of Csk is needed for T-cell activation to proceed. Within the same time course, a Csk-G3BP complex is formed and seems to sequester Csk outside lipid rafts (see Mosenden R. and Tasken K., 2011, ibid, and Tasken K. and Ruppelt A., 2006, ibid).

In contrast, combined TCR and CD28 stimulation mediates recruitment of the cyclic nucleotide phosphodiesterase PDE4 to lipid rafts, which enhances cAMP degradation (see Fig. 6 of Abrahamsen H. et al., 2004, ibid). As such, TCR induced production of cAMP is countered, and the T-cell immune response potentiated. Upon TCR stimulation alone, PDE4 recruitment may be too low to fully reduce the cAMP levels and therefore maximal T-cell activation cannot occur (see Abrahamsen H. et al., 2004, ibid).

Thus, by active suppression of proximal TCR signalling, signalling via cAMP-PKA-Csk is thought to set the threshold for T-cell activation. Recruitment of PDEs can counter this suppression. Tissue or cell-type specific regulation is accomplished through expression of multiple isoforms of AC, PKA, and PDEs. As mentioned above, the balance between activation and suppression needs to be tightly regulated to prevent development of autoimmune disorders, immune deficiencies and cancer.

The identification and role of the T-Cell receptor signalling genes has been described in WO 2021/175986 hereby incorporated by reference in its entirety.

Selection of the genes

Gene signatures, and combinations of these signatures with clinical parameters, have been identified of which the resulting models show a significant relation to mortality and therefore are expected to improve the prediction of the effectiveness of these treatments.

The identified immune defense response genes AIM2, APOBEC3A, CIAO1, DDX58, DHX9, IFI16, IFIH1, IFIT1, IFIT3, LRRFIP1, MYD88, OAS1, TLR8, and ZBP1, respectively, were identified as follows: A group of 538 prostate cancer patients were treated with RP and the prostate cancer tissue was stored together with clinical (e.g. pathological Gleason grade group (pGGG), pathology state (pT stage)) as well as relevant outcome parameters (e.g. biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis , which resulted in a range of enriched annotation clusters. The annotation cluster #2 demonstrated enrichment (enrichment score: 10.8) in 30 genes with a function in defense response to viruses, negative regulation of viral genome replication as well as type I interferon signalling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signalling was further enriched to 61 genes by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The identified T-Cell receptor signalling genes CD2, CD247, CD28, CD3E, CD3G, CD4, CSK, EZR, FYN, LAT, LCK, PAG1, PDE4D, PRKACA, PRKACB, PTPRC, and ZAP70 were identified as follows: A group of 538 prostate cancer patients were treated with RP and the prostate cancer tissue was stored together with clinical (e.g. pathological Gleason grade group (pGGG), pathology state (pT stage)) as well as relevant outcome parameters (e.g. biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis, which resulted in a range of enriched annotation clusters. The annotation cluster #6 demonstrated enrichment (enrichment score: 5.9) in 17 genes with a function in primary immune deficiency and activation of T-Cell receptor signalling. A further heat map analysis confirmed that these T-Cell receptor signalling genes were generally higher expressed in samples from patients in PDE4D7 score class 1 than from patients in PDE4D7 score class 4.

The identified PDE4D7 correlated genes ABCC5, CUX2, KIAA1549, PDE4D, RAP1GAP2, SLC39A11, TDRD1, and VWA2 were identified as follows: In RNAseq data generated on 571 prostate cancer patients on close to 60,000 transcripts a range of genes were identified that are correlated to the expression of the known biomarker PDE4D7 in this data. The correlation between the expression of any of these genes and PDE4D7 across the 571 samples was done by Pearson correlation and is expressed as a value between 0 to 1 in case of positive correlation or a value between -1 to 0 in case of negative correlation. As input data for the calculation of the correlation coefficient the PDE4D7 score (see Alves de Inda M. et al., 2018, ibid) and the RNAseq determined TPM gene expression value per gene of interest (see below) were used.

The maximum negative correlation coefficient identified between the expression of any of the approximately 60,000 transcripts and the expression of PDE4D7 was -0.38 while the maximum positive correlation coefficient identified between the expression of any of the approximately 60,000 transcripts and the expression of PDE4D7 was +0.56. Genes in the range of correlation -0.31 to -0.38 as well as +0.41 to +0.56 were selected. In total 77 transcripts matching these characteristics were identified. From those 77 transcripts the eight PDE4D7 correlated genes ABCC5, CUX2, KIAA1549, PDE4D, RAP1GAP2, SLC39A11, TDRD1, and VWA2 were selected by testing Cox regression combination models iteratively in a sub-cohort of 186 patients who were undergoing salvage radiation treatment (SRT) due to post-surgical biochemical relapse. The clinical endpoint tested was prostate cancer specific death after start of SRT. The boundary condition for the selection of the eight genes was given by the restriction that the p-values in the multivariate Cox-regression were <0.1 for all genes retained in the model.

In this document, it is shown that the PDE4D7 correlated genes are also of predictive value with respect to an outcome of a prostate cancer subject, preferably an outcome of an androgen deprivation therapy. As the set of PDE4D7 correlated are identified as specifically responding to PDE4D7 expression levels, it is further theorized that PDE4D7 expression levels per se have the same predictive effect.

Therefore, in a first embodiment the invention provides for a method of predicting a response of a prostate cancer subject to androgen deprivation therapy, comprising:

- determining or receiving the result of a determination of a gene expression profile comprising: the gene expression level of PDE4D7, and/or
  three or more gene expression levels, wherein the three or more gene expression levels are selected from:

  - PDE4D7 correlated genes selected from the group consisting of: ABCC5, CUX2, KIAA1549, PDE4D, RAP1GAP2, SLC39A11, TDRD1, and VWA2,

  said gene expression profile being determined in a biological sample obtained from the subject,
- determining the prediction of the outcome based on the gene expression profile,
- wherein said prediction is a favourable or a non-favourable response to androgen deprivation therapy.

Optionally, the method further comprises a step of providing the prediction of the outcome to a medical caregiver or the subject.

In an embodiment the invention provides for a computer implemented method of predicting a response of a prostate cancer subject to androgen deprivation therapy, comprising:

- receiving the result of a determination of a gene expression profile comprising the gene expression level of PDE4D7, and/or three or more gene expression levels, wherein the three or more gene expression levels are selected from:

  - PDE4D7 correlated genes selected from the group consisting of: ABCC5, CUX2, KIAA1549, PDE4D, RAP1GAP2, SLC39A11, TDRD1, and VWA2,

  said gene expression profile being determined in a biological sample obtained from the subject,

- determining the prediction of the outcome based on the gene expression profile,
- wherein said prediction is a favourable or a non-favourable response to androgen deprivation therapy. Optionally, the method further comprises a step of providing the prediction of the outcome to a medical caregiver or the subject.

In an alternative embodiment the invention relates to a method of treating a subject with prostate cancer, the method comprising:

- determining or receiving the result of a determination of a gene expression profile comprising: the gene expression level of PDE4D7, and/or

three or more gene expression levels, wherein the three or more gene expression levels are selected from:

- PDE4D7 correlated genes selected from the group consisting of: ABCC5, CUX2, KIAA1549, PDE4D, RAP1GAP2, SLC39A11, TDRD1, and VWA2,

said gene expression profile being determined in a biological sample obtained from the subject,
- determining the prediction of the outcome based on the gene expression profile,
- wherein said prediction is a favourable or a non-favourable response to androgen deprivation therapy,

wherein if the prediction is favourable androgen deprivation therapy is administered to the subject prior to locoregional therapy; and
if the prediction is not favourable androgen deprivation therapy is not administered the subject prior to locoregional therapy. In an embodiment the locoregional therapy is radical prostatectomy (RP) or radiation therapy (RT).

In an embodiment the gene expression profile comprises the PDE4D7 expression level and one or more, for example one, two, three, four, five, six, seven or all of the PDE4D7 correlated genes selected from ABCC5, CUX2, KIAA1549, PDE4D, RAP1GAP2, SLC39A11, TDRD1, and VWA2. In an embodiment the gene expression profile comprises three or more, for example three, four, five, six, seven or all of the PDE4D7 correlated genes selected from ABCC5, CUX2, KIAA1549, PDE4D, RAP1GAP2, SLC39A11, TDRD1, and VWA2.

It is theorized that the model may be improved by including one or more expression level from the set of immune defense response genes and/or T-Cell receptor signaling genes. Therefore, in a further embodiment the gene expression profile further comprises one or more gene expression level selected from:

- immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
- T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70.

In the present invention prostate cancer related death preferably is prostate-cancer specific death.

With respect to the biological processes described above, three immune system related gene signatures were selected, including the genes listed in tables 1-3. Before, the relevance of these signatures to prediction of prostate cancer survival was shown.

**Table 1:** Immune Defence Response (IDR) Signature.

| Gene Symbol | Ensembl_ID |
|---|---|
| AIM2 | ENSG00000163568 |
| APOBEC3A | ENSG00000128383 |
| CIAO1 | ENSG00000 144021 |
| DDX58 | ENSG00000 107201 |
| DHX9 | ENSG00000135829 |
| IFI16 | ENSG00000163565 |
| IFIH1 | ENSG00000115267 |
| IFIT1 | ENSG00000185745 |

(continued)

| Gene Symbol | Ensembl_ID |
|---|---|
| IFIT3 | ENSG00000 119917 |
| LRRFIP1 | ENSG00000 124831 |
| MYD88 | ENSG00000172936 |
| OAS1 | ENSG00000089127 |
| TLR8 | ENSG00000 101916 |
| ZBP1 | ENSG00000124256 |

**Table 2:** T-Cell Receptor (TCR) Signature.

| Gene Symbol | Ensembl_ID |
|---|---|
| CD2 | ENSG00000 116824 |
| CD247 | ENSG00000198821 |
| CD28 | ENSG00000178562 |
| CD3E | ENSG00000198851 |
| CD3G | ENSG00000160654 |
| CD4 | ENSG00000010610 |
| CSK | ENSG00000103653 |
| EZR | ENSG00000092820 |
| FYN | ENSG00000010810 |
| LAT | ENSG00000213658 |
| LCK | ENSG00000182866 |
| PAG1 | ENSG00000076641 |
| PDE4D | ENSG00000113448 |
| PRKACA | ENSG00000072062 |
| PRKACB | ENSG00000142875 |
| PTPRC | ENSG00000081237 |
| ZAP70 | ENSG00000115085 |

**Table 3:** PDE4D7 Correlated (PDE4D7_R2) signature.

| Gene Symbol | Ensembl_ID |
|---|---|
| ABCC5 | ENSG00000114770 |
| CUX2 | ENSG00000111249 |
| KIAA1549 | ENSG00000122778 |
| PDE4D | ENSG00000113448 |
| RAP1GAP2 | ENSG00000132359 |
| SLC39A11 | ENSG00000133195 |
| TDRD1 | ENSG00000095627 |
| VWA2 | ENSG00000165816 |

It was found that the genes AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70, ABCC5, CUX2, KIAA1549, PDE4D, RAP1GAP2, SLC39A11, TDRD1, and VWA2 each individually, one or more per gene panel, in a combination of one or more per gene panel or when all combined, are able to predict the outcome in a prostate cancer subject.

The term "ABCC5" refers to the human ATP binding cassette subfamily C member 5 gene (Ensembl: ENSG00000114770), for example, to the sequence as defined in NCBI Reference Sequence NM_001023587.2 or in NCBI Reference Sequence NM_005688.3, specifically, to the nucleotide sequence as set forth in SEQ ID NO: 1 or in SEQ ID NO:2, which correspond to the sequences of the above indicated NCBI Reference Sequences of the ABCC5 transcript, and also relates to the corresponding amino acid sequence for example as set forth in SEQ ID NO:3 or in SEQ ID NO:4, which correspond to the protein sequences defined in NCBI Protein Accession Reference Sequence NP_001018881.1 and in NCBI Protein Accession Reference Sequence NP_005679 encoding the ABCC5 polypeptide.

The term "ABCC5" also comprises nucleotide sequences showing a high degree of homology to ABCC5, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2.

The term "AIM2" refers to the Absent in Melanoma 2 gene (Ensembl: ENSG00000163568), for example, to the sequence as defined in NCBI Reference Sequence NM_004833, specifically, to the nucleotide sequence as set forth in SEQ ID NO:5, which corresponds to the sequence of the above indicated NCBI Reference Sequence of the AIM2 transcript, and also relates to the corresponding amino acid sequence for example as set forth in SEQ ID NO:6, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g. nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:7, which corresponds to the sequence of the above indicated NCBI Reference Sequence of the APOBEC3A transcript, and also relates to the corresponding amino acid sequence for example as set forth in SEQ ID NO:8, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g. nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "CD2" refers to the Cluster Of Differentiation 2 gene (Ensembl: ENSG00000116824), for example, to the sequence as defined in NCBI Reference Sequence NM_001767, specifically, to the nucleotide sequence as set forth in SEQ ID NO:9, which corresponds to the sequence of the above indicated NCBI Reference Sequence of the CD2 transcript, and also relates to the corresponding amino acid sequence for example as set forth in SEQ ID NO:10, which corresponds to the protein sequence defined in NCBI Protein Accession Reference Sequence NP_001758 encoding the CD2 polypeptide.

The term "CD2" also comprises nucleotide sequences showing a high degree of homology to CD2, e.g. nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "CD247" refers to the Cluster Of Differentiation 247 gene (Ensembl: ENSG00000198821), for example, to the sequence as defined in NCBI Reference Sequence NM_000734 or in NCBI Reference Sequence NM_198053, specifically, to the nucleotide sequence as set forth in SEQ ID NO:11 or in SEQ ID NO:12, which correspond to the sequences of the above indicated NCBI Reference Sequences of the CD247 transcript, and also relates to the corresponding amino acid sequence for example as set forth in SEQ ID NO:13 or in SEQ ID NO:14, which correspond to the protein sequences defined in NCBI Protein Accession Reference Sequence NP_000725 and in NCBI Protein Accession Reference Sequence NP_932170 encoding the CD247 polypeptide.

The term "CD247" also comprises nucleotide sequences showing a high degree of homology to CD247, e.g. nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or amino acid sequences being at least 75%, 80%, 85%, 90%, 91%, 92%, 93%, 94%, 95%, 96%, 97%, 98% or 99% identical to the sequence as set forth in SEQ ID NO:13 or in SEQ ID NO:14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:13 or in SEQ ID NO:14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO: 12.

The term "CD28" refers to the Cluster Of Differentiation 28 gene (Ensembl: ENSG00000178562), for example, to the sequence as defined in NCBI Reference Sequence NM_006139 or in NCBI Reference Sequence NM_001243078, specifically, to the nucleotide sequence as set forth in SEQ ID NO: 15 or in SEQ ID NO: 16, which correspond to the sequences of the above indicated NCBI Reference Sequences of the CD28 transcript, and also relates to the corresponding amino acid sequence for example as set forth in SEQ ID NO:17 or in SEQ ID NO: 18, which correspond to the protein sequences defined in NCBI Protein Accession Reference Sequence NP_006130 and in NCBI Protein Accession Reference Sequence NP_001230007 encoding the CD28 polypeptide.

The term "CD28" also comprises nucleotide sequences showing a high degree of homology to CD28, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO:16 or amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:17 or in SEQ ID NO:18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16.

The term "CD3E" refers to the Cluster Of Differentiation 3E gene (Ensembl: ENSG00000198851), for example, to the sequence as defined in NCBI Reference Sequence NM_000733, specifically, to the nucleotide sequence as set forth in SEQ ID NO: 19, which corresponds to the sequence of the above indicated NCBI Reference Sequence of the CD3E transcript, and also relates to the corresponding amino acid sequence for example as set forth in SEQ ID NO:20, which corresponds to the protein sequence defined in NCBI Protein Accession Reference Sequence NP_000724 encoding the CD3E polypeptide.

The term "CD3E" also comprises nucleotide sequences showing a high degree of homology to CD3E, e.g. nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19.

The term "CD3G" refers to the Cluster Of Differentiation 3G gene (Ensembl: ENSG00000160654), for example, to the sequence as defined in NCBI Reference Sequence NM_000073, specifically, to the nucleotide sequence as set forth in SEQ ID NO:21, which corresponds to the sequence of the above indicated NCBI Reference Sequence of the CD3G transcript, and also relates to the corresponding amino acid sequence for example as set forth in SEQ ID NO:22, which corresponds to the protein sequence defined in NCBI Protein Accession Reference Sequence NP_000064 encoding the CD3G polypeptide.

The term "CD3G" also comprises nucleotide sequences showing a high degree of homology to CD3G, e.g.

nucleic acid sequences being at least 75%, 80%, 85%, 90%, 91%, 92%, 93%, 94%, 95%, 96%, 97%, 98% or 99% identical to the sequence as set forth in SEQ ID NO:21 or amino acid sequences being at least 75%, 80%, 85%, 90%, 91%, 92%, 93%, 94%, 95%, 96%, 97%, 98% or 99% identical to the sequence as set forth in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21.

The term "CD4" refers to the Cluster Of Differentiation 4 gene (Ensembl: ENSG00000010610), for example, to the sequence as defined in NCBI Reference Sequence NM_000616, specifically, to the nucleotide sequence as set forth in SEQ ID NO:23, which corresponds to the sequence of the above indicated NCBI Reference Sequence of the CD4 transcript, and also relates to the corresponding amino acid sequence for example as set forth in SEQ ID NO:24, which corresponds to the protein sequence defined in NCBI Protein Accession Reference Sequence NP_000607 encoding the CD4 polypeptide.

The term "CD4" also comprises nucleotide sequences showing a high degree of homology to CD4, e.g. nucleic acid sequences being at least 75%, 80%, 85%, 90%, 91%, 92%, 93%, 94%, 95%, 96%, 97%, 98% or 99% identical to the sequence as set forth in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:23.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:25, which corresponds to the sequence of the above indicated NCBI Reference Sequence of the CIAO1 transcript, and also relates to the corresponding amino acid sequence for example as set forth in SEQ ID NO:26, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:25 or amino acid sequences being at least 75%, 80%, 85%, 90%, 91%, 92%, 93%, 94%, 95%, 96%, 97%, 98% or 99% identical to the sequence as set forth in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25.

The term "CSK" refers to the C-Terminal Src Kinase gene (Ensembl: ENSG00000103653), for example, to the sequence as defined in NCBI Reference Sequence NM_004383, specifically, to the nucleotide sequence as set forth in SEQ ID NO:27, which corresponds to the sequence of the above indicated NCBI Reference Sequence of the CSK transcript, and also relates to the corresponding amino acid sequence for example as set forth in SEQ ID NO:28, which corresponds to the protein sequence defined in NCBI Protein Accession Reference Sequence NP_004374 encoding the CSK polypeptide.

The term "CSK" also comprises nucleotide sequences showing a high degree of homology to CSK, e.g. nucleic acid sequences being at least 75%, 80%, 85%, 90%, 91%, 92%, 93%, 94%, 95%, 96%, 97%, 98% or 99% identical to the sequence as set forth in SEQ ID NO:27 or amino acid sequences being at least 75%, 80%, 85%, 90%, 91%, 92%, 93%, 94%, 95%, 96%, 97%, 98% or 99% identical to the sequence as set forth in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27.

The term "CUX2" refers to the human Cut Like Homeobox 2 gene (Ensembl: ENSG00000111249), for example, to the sequence as defined in NCBI Reference Sequence NM_015267.3, specifically, to the nucleotide sequence as set forth in SEQ ID NO:29, which corresponds to the sequence of the above indicated NCBI Reference Sequence of the CUX2 transcript, and also relates to the corresponding amino acid sequence for example as set forth in SEQ ID NO:30, which corresponds to the protein sequence defined in NCBI Protein Accession Reference Sequence NP_056082.2 encoding the CUX2 polypeptide.

The term "CUX2" also comprises nucleotide sequences showing a high degree of homology to CUX2, e.g. nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:31, which corresponds to the sequence of the above indicated NCBI Reference Sequence of the DDX58 transcript, and also relates to the corresponding amino acid sequence for example as set forth in SEQ ID NO:32, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g. nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:33, which corresponds to the sequence of the above indicated NCBI Reference Sequence of the DHX9 transcript, and also relates to the corresponding amino acid sequence for example as set forth in SEQ ID NO:34, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g. nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "EZR" refers to the Ezrin gene (Ensembl: ENSG00000092820), for example, to the sequence as defined in NCBI Reference Sequence NM_003379, specifically, to the nucleotide sequence as set forth in SEQ ID NO:35, which corresponds to the sequence of the above indicated NCBI Reference Sequence of the EZR transcript, and also relates to the corresponding amino acid sequence for example as set forth in SEQ ID NO:36, which corresponds to the protein sequence defined in NCBI Protein Accession Reference Sequence NP_003370 encoding the EZR polypeptide.

The term "EZR" also comprises nucleotide sequences showing a high degree of homology to EZR, e.g. nucleic acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being at least 75%, 80%, 85%, 90%, 91%, 92%, 93%, 94%, 95%, 96%, 97%, 98% or 99% identical to the sequence as set forth in SEQ ID NO:36 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35.

The term "FYN" refers to the FYN Proto-Oncogene gene (Ensembl: ENSG00000010810), for example, to the sequence as defined in NCBI Reference Sequence NM_002037 or in NCBI Reference Sequence NM_153047 or in NCBI Reference Sequence NM_153048, specifically, to the nucleotide sequence as set forth in SEQ ID NO:37 or in SEQ ID NO:38 or in SEQ ID NO:39, which correspond to the sequences of the above indicated NCBI Reference Sequences of the FYN transcript, and also relates to the corresponding amino acid sequence for example as set forth in SEQ ID NO:40 or in SEQ ID NO:41 or in SEQ ID NO:42, which correspond to the protein sequences defined in NCBI Protein Accession Reference Sequence NP_002028 and in NCBI Protein Accession Reference Sequence NP_694592 and in NCBI Protein Accession Reference Sequence XP_005266949 encoding the FYN polypeptide.

The term "FYN" also comprises nucleotide sequences showing a high degree of homology to FYN, e.g. nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:43, which corresponds to the sequence of the above indicated NCBI Reference Sequence of the IFI16 transcript, and also relates to the corresponding amino acid sequence for example as set forth in SEQ ID NO:44, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g. nucleic acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:43.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO:45, which corresponds to the sequence of the above indicated NCBI Reference Sequence of the IFIH1 transcript, and also relates to the corresponding amino acid sequence for example as set forth in SEQ ID NO:46, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being at least 75%, 80%, 85%, 90%, 91%, 92%, 93%, 94%, 95%, 96%, 97%, 98% or 99% identical to the sequence as set forth in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:45.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO:47 or in SEQ ID NO:48, which correspond to the sequences of the above indicated NCBI Reference Sequences of the **IFIT1** transcript, and also relates to the corresponding amino acid sequence for example as set forth in SEQ ID NO:49 or in SEQ ID NO:50, which correspond to the protein sequences defined in NCBI Protein Accession Reference Sequence NP_001257858 and in NCBI Protein Accession Reference Sequence NP_001539 encoding the **IFIT1** polypeptide.

The term "IFIT1" also comprises nucleotide sequences showing a high degree of homology to IFIT1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO:51, which corresponds to the sequence of the above indicated NCBI Reference Sequence of the IFIT3 transcript, and also relates to the corresponding amino acid sequence for example as set forth in SEQ ID NO:52, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g. nucleic acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being at least 75%, 80%, 85%, 90%, 91%, 92%, 93%, 94%, 95%, 96%, 97%, 98% or 99% identical to the sequence as set forth in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51.

The term "KIAA1549" refers to the human KIAA1549 gene (Ensembl: ENSG00000122778), for example, to the sequence as defined in NCBI Reference Sequence NM_020910 or in NCBI Reference Sequence NM_001164665, specifically, to the nucleotide sequence as set forth in SEQ ID NO:53 or in SEQ ID NO:54, which correspond to the sequences of the above indicated NCBI Reference Sequence of the KIAA1549 transcript, and also relates to the corresponding amino acid sequence for example as set forth in SEQ ID NO:55 or in SEQ ID NO:56, which correspond to the protein sequences defined in NCBI Protein Accession Reference Sequence NP_065961 and in NCBI Protein Accession Reference Sequence NP_001158137 encoding the KIAA1549 polypeptide.

The term "KIAA1549" also comprises nucleotide sequences showing a high degree of homology to KIAA1549, e.g. nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54 or amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54.

The term "LAT" refers to the Linker For Activation Of T-Cells gene (Ensembl: ENSG00000213658), for example, to the sequence as defined in NCBI Reference Sequence NM_001014987 or in NCBI Reference Sequence NM_014387, specifically, to the nucleotide sequence as set forth in SEQ ID NO:57 or in SEQ ID NO:58, which correspond to the sequences of the above indicated NCBI Reference Sequences of the LAT transcript, and also relates to the corresponding amino acid sequence for example as set forth in SEQ ID NO:59 or in SEQ ID NO:60, which corresponds to the protein sequences defined in NCBI Protein Accession Reference Sequence NP_001014987 and in NCBI Protein Accession Reference Sequence NP_055202 encoding the LAT polypeptide.

The term "LAT" also comprises nucleotide sequences showing a high degree of homology to LAT, e.g. nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58 or amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58.

The term "LCK" refers to the LCK Proto-Oncogene gene (Ensembl: ENSG00000182866), for example, to the sequence as defined in NCBI Reference Sequence NM_005356, specifically, to the nucleotide sequence as set forth in SEQ ID NO:61, which corresponds to the sequence of the above indicated NCBI Reference Sequence of the LCK transcript, and also relates to the corresponding amino acid sequence for example as set forth in SEQ ID NO:62, which corresponds to the protein sequence defined in NCBI Protein Accession Reference Sequence NP_005347 encoding the LCK polypeptide.

The term "LCK" also comprises nucleotide sequences showing a high degree of homology to LCK, e.g. nucleic acid sequences being at least 75%, 80%, 85%, 90%, 91%, 92%, 93%, 94%, 95%, 96%, 97%, 98% or 99% identical to the sequence as set forth in SEQ ID NO:61 or amino acid sequences being at least 75%, 80%, 85%, 90%, 91%, 92%, 93%, 94%, 95%, 96%, 97%, 98% or 99% identical to the sequence as set forth in SEQ ID NO:62 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:62 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:61.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:63 or in SEQ ID NO:64 or in SEQ ID NO:65 or in SEQ ID NO:66, which correspond to the sequences of the above indicated NCBI Reference Sequences of the LRRFIP1 transcript, and also relates to the corresponding amino acid sequence for example as set forth in SEQ ID NO:67 or in SEQ ID NO:68 or in SEQ ID NO:69 or in SEQ ID NO:70, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66 or amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:71 or in SEQ ID NO:72 or in SEQ ID NO:73 or in SEQ ID NO:74 or in SEQ ID NO:75, which correspond to the sequences of the above indicated NCBI Reference Sequences of the MYD88 transcript, and also relates to the corresponding amino acid sequence for example as set forth in SEQ ID NO:76 or in SEQ ID NO:77 or in SEQ ID NO:78 or in SEQ ID NO:79 or in SEQ ID NO:80, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g. nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75 or amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75.

The term "OAS1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:81 or in SEQ ID NO:82 or in SEQ ID NO:83 or in SEQ ID NO:84, which correspond to the sequences of the above indicated NCBI Reference Sequences of the OAS1 transcript, and also relates to the corresponding amino acid sequences for example as set forth in SEQ ID NO:85 or in SEQ ID NO:86 or in SEQ ID NO:87 or in SEQ ID NO:88, which correspond to the protein sequences defined in NCBI Protein Accession Reference Sequence NP_001307080 and in NCBI Protein Accession Reference Sequence NP_002525 and in NCBI Protein Accession Reference Sequence NP_001027581 and in NCBI Protein Accession Reference Sequence NP_058132 encoding the OAS1 polypeptide.

The term "OAS1" also comprises nucleotide sequences showing a high degree of homology to OAS1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84 or amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84.

The term "PAG1" refers to the Phosphoprotein Membrane Anchor With Glycosphingolipid Microdomains 1 gene (Ensembl: ENSG00000076641), for example, to the sequence as defined in NCBI Reference Sequence NM_018440, specifically, to the nucleotide sequence as set forth in SEQ ID NO:89, which corresponds to the sequence of the above indicated NCBI Reference Sequence of the **PAG1** transcript, and also relates to the corresponding amino acid sequence for example as set forth in SEQ ID NO:90, which corresponds to the protein sequence defined in NCBI Protein Accession Reference Sequence NP_060910 encoding the **PAG1** polypeptide.

The term "PAG1" also comprises nucleotide sequences showing a high degree of homology to PAG1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:89 or amino acid sequences being at least 75%, 80%, 85%, 90%, 91%, 92%, 93%, 94%, 95%, 96%, 97%, 98% or 99% identical to the sequence as set forth in SEQ ID NO:90 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:90 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:89.

The term "PDE4D" refers to the human Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001165899, specifically, to the nucleotide sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99, which correspond to the sequences of the above indicated NCBI Reference Sequence of the PDE4D transcript, and also relates to the corresponding amino acid sequence for example as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g. nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99 or amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99.

The term "PRKACA" refers to the Protein Kinase cAMP-Activated Catalytic Subunit Alpha gene (Ensembl: ENSG00000072062), for example, to the sequence as defined in NCBI Reference Sequence NM_002730 or in NCBI Reference Sequence NM_207518, specifically, to the nucleotide sequences as set forth in SEQ ID NO:109 or in SEQ ID NO:110, which correspond to the sequences of the above indicated NCBI Reference Sequences of the PRKACA transcript, and also relates to the corresponding amino acid sequence for example as set forth in SEQ ID NO:111 or in SEQ ID NO:112, which correspond to the protein sequences defined in NCBI Protein Accession Reference Sequence NP_002721 and in NCBI Protein Accession Reference Sequence NP_997401 encoding the PRKACA polypeptide.

The term "PRKACA" also comprises nucleotide sequences showing a high degree of homology to PRKACA, e.g. nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110 or amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110.

The term "PRKACB" refers to the Protein Kinase cAMP-Activated Catalytic Subunit Beta gene (Ensembl:

ENSG00000142875), for example, to the sequence as defined in NCBI Reference Sequence NM_002731 or in NCBI Reference Sequence NM_182948 or in NCBI Reference Sequence NM_001242860 or in NCBI Reference Sequence NM_001242859 or in NCBI Reference Sequence NM_001242858 or in NCBI Reference Sequence NM_001242862 or in NCBI Reference Sequence NM_001242861 or in NCBI Reference Sequence NM_001300915 or in NCBI Reference Sequence NM_207578 or in NCBI Reference Sequence NM_001242857 or in NCBI Reference Sequence NM_001300917, specifically, to the nucleotide sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123, which correspond to the sequences of the above indicated NCBI Reference Sequences of the PRKACB transcript, and also relates to the corresponding amino acid sequence for example as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134, which correspond to the protein sequences defined in NCBI Protein Accession Reference Sequence NP_002722 and in NCBI Protein Accession Reference Sequence NP_891993 and in NCBI Protein Accession Reference Sequence NP_001229789 and in NCBI Protein Accession Reference Sequence NP_001229788 and in NCBI Protein Accession Reference Sequence NP_001229787 and in NCBI Protein Accession Reference Sequence NP_001229791 and in NCBI Protein Accession Reference Sequence NP_001229790 and in NCBI Protein Accession Reference Sequence NP_001287844 and in NCBI Protein Accession Reference Sequence NP_997461 and in NCBI Protein Accession Reference Sequence NP_001229786 and in NCBI Protein Accession Reference Sequence NP_001287846 encoding the PRKACB polypeptide.

The term "PRKACB" also comprises nucleotide sequences showing a high degree of homology to PRKACB, e.g. nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123 or amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123.

The term "PTPRC" refers to the Protein Tyrosine Phosphatase Receptor Type C gene (Ensembl: ENSG00000081237), for example, to the sequence as defined in NCBI Reference Sequence NM_002838 or in NCBI Reference Sequence NM_080921, specifically, to the nucleotide sequence as set forth in SEQ ID NO:135 or in SEQ ID NO:136, which correspond to the sequences of the above indicated NCBI Reference Sequences of the PTPRC transcript, and also relates to the corresponding amino acid sequence for example as set forth in SEQ ID NO:137 or in SEQ ID NO:138, which correspond to the protein sequences defined in NCBI Protein Accession Reference Sequence NP_002829 encoding the PTPRC polypeptide and in NCBI Protein Accession Reference Sequence NP_563578.

The term "PTPRC" also comprises nucleotide sequences showing a high degree of homology to PTPRC, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 135 or in SEQ ID NO:136 or amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO: 138 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 137 or in SEQ ID NO:138 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 135 or in SEQ ID NO: 136.

The term "RAP1GAP2" refers to the human RAP1 GTPase Activating Protein 2 gene (ENSG00000132359), for example, to the sequence as defined in NCBI Reference Sequence NM_015085 or in NCBI Reference Sequence NM_001100398 or in NCBI Reference Sequence NM_001330058, specifically, to the nucleotide sequence as set forth in SEQ ID NO: 139 or in SEQ ID NO: 140 or in SEQ ID NO:141, which correspond to the sequences of the above indicated NCBI Reference Sequences of the **RAP1GAP2** transcript, and also relates to the corresponding amino acid sequence for example as set forth in SEQ ID NO: 142 or in SEQ ID NO:143 or in SEQ ID NO:144, which correspond to the protein sequences defined in NCBI Protein Accession Reference Sequence NP_055900 and in NCBI Protein Accession Reference Sequence NP_001093868 and in NCBI Protein Accession Reference Sequence NP_001316987 encoding the **RAP1GAP2** polypeptide.

The term "RAP1GAP2" also comprises nucleotide sequences showing a high degree of homology to RAP1GAP2, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 139 or in SEQ ID NO:140 or in SEQ ID NO:141 or amino acid sequences being at least 75%, 80%, 85%, 90%, 91%, 92%, 93%, 94%, 95%, 96%, 97%, 98% or 99% identical to the sequence as set forth in SEQ ID NO: 142 or in SEQ ID NO:143 or in SEQ ID NO:144 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 142 or in SEQ ID NO:143 or in SEQ ID NO: 144 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 139 or in SEQ ID NO:140 or in SEQ ID NO: 141.

The term "SLC39A11" refers to the human Solute Carrier Family 39 Member 11 gene (Ensembl: ENSG00000133195), for example, to the sequence as defined in NCBI Reference Sequence NM_139177 or in NCBI Reference Sequence NM_001352692, specifically, to the nucleotide sequence as set forth in SEQ ID NO:145 or in SEQ ID NO:146, which correspond to the sequences of the above indicated NCBI Reference Sequences of the **SLC39A11** transcript, and also relates to the corresponding amino acid sequence for example as set forth in SEQ ID NO:147 or in SEQ ID NO: 148, which correspond to the protein sequences defined in NCBI Protein Accession Reference Sequence NP_631916 and in NCBI Protein Accession Reference Sequence NP_001339621 encoding the **SLC39A11** polypeptide.

The term "SLC39A11" also comprises nucleotide sequences showing a high degree of homology to SLC39A11, e.g. nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO:146 or amino acid sequences being at least 75%, 80%, 85%, 90%, 91%, 92%, 93%, 94%, 95%, 96%, 97%, 98% or 99% identical to the sequence as set forth in SEQ ID NO:147 or in SEQ ID NO: 148 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 147 or in SEQ ID NO:148 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO: 146.

The term "TDRD1" refers to the human Tudor Domain Containing 1 gene (Ensembl: ENSG00000095627), for example, to the sequence as defined in NCBI Reference Sequence NM_198795, specifically, to the nucleotide sequence as set forth in SEQ ID NO: 149, which corresponds to the sequence of the above indicated NCBI Reference Sequence of the **TDRD1** transcript, and also relates to the corresponding amino acid sequence for example as set forth in SEQ ID NO: 150, which corresponds to the protein sequence defined in NCBI Protein Accession Reference Sequence NP_942090 encoding the **TDRD1** polypeptide.

The term "TDRD1" also comprises nucleotide sequences showing a high degree of homology to TDRD1, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 149 or amino acid sequences being at least 75%, 80%, 85%, 90%, 91%, 92%, 93%, 94%, 95%, 96%, 97%, 98% or 99% identical to the sequence as set forth in SEQ ID NO: 150 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 150 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 149.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:151 or in SEQ ID NO:152, which correspond to the sequences of the above indicated NCBI Reference Sequences of the TLR8 transcript, and also relates to the corresponding amino acid sequences for example as set forth in SEQ ID NO: 153 or in SEQ ID NO:154, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 151 or in SEQ ID NO:152 or amino acid sequences being at least 75%, 80%, 85%, 90%, 91%, 92%, 93%, 94%, 95%, 96%, 97%, 98% or 99% identical to the sequence as set forth in SEQ ID NO:153 or in SEQ ID NO:154 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 153 or in SEQ ID NO: 154 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 151 or in SEQ ID NO: 152.

The term "VWA2" refers to the human Von Willebrand Factor A Domain Containing 2 gene (Ensembl: ENSG00000165816), for example, to the sequence as defined in NCBI Reference Sequence NM_001320804, specifically, to the nucleotide sequence as set forth in SEQ ID NO:155, which corresponds to the sequence of the above indicated NCBI Reference Sequence of the VWA2 transcript, and also relates to the corresponding amino acid sequence for example as set forth in SEQ ID NO:156, which corresponds to the protein sequence defined in NCBI Protein Accession Reference Sequence NP_001307733 encoding the VWA2 polypeptide.

The term "VWA2" also comprises nucleotide sequences showing a high degree of homology to VWA2, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 155 or amino acid sequences being at least 75%, 80%, 85%, 90%, 91%, 92%, 93%, 94%, 95%, 96%, 97%, 98% or 99% identical to the sequence as set forth in SEQ ID NO: 156 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 156 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 155.

The term "ZAP70" refers to the Zeta Chain Of T-Cell Receptor Associated Protein Kinase 70 gene (Ensembl: ENSG00000115085), for example, to the sequence as defined in NCBI Reference Sequence NM_001079 or in NCBI Reference Sequence NM_207519, specifically, to the nucleotide sequences as set forth in SEQ ID NO: 157 or in SEQ ID NO:158, which correspond to the sequences of the above indicated NCBI Reference Sequences of the ZAP70 transcript, and also relates to the corresponding amino acid sequence for example as set forth in SEQ ID NO: 159 or in SEQ ID NO:160, which corresponds to the protein sequences defined in NCBI Protein Accession Reference Sequence NP_001070 and in NCBI Protein Accession Reference Sequence NP_997402 encoding the ZAP70 polypeptide.

The term "ZAP70" also comprises nucleotide sequences showing a high degree of homology to ZAP70, e.g. nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158or amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:161 or in SEQ ID NO:162 or in SEQ ID NO:163, which corresponds to the sequence of the above indicated NCBI Reference Sequence of the ZBP1 transcript, and also relates to the corresponding amino acid sequence for example as set forth in SEQ ID NO:164 or in SEQ ID NO:165 or in SEQ ID NO:166, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163 or amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163.

The term "PDE4D7" refers to the phosphodiesterase 4D isoform 7 gene (Ensembl: ENSG00000113448), for example, to the sequence as defined in NCBI Reference Sequence NM_001364599, specifically, to the nucleotide sequence as set forth in SEQ ID NO:167, which corresponds to the sequence of the above indicated NCBI Reference Sequence of the PDE4D7 transcript, and also relates to the corresponding amino acid sequence for example as set forth in SEQ ID NO: 168, which corresponds to the protein sequences defined in NCBI Protein Accession Reference Sequence NP_001351528 encoding the PDE4D7 polypeptide.

The term "PDE4D7" also comprises nucleotide sequences showing a high degree of homology to PDE4D7, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 167 or amino acid sequences being at least 75%, 80%, 85%, 90%, 91%, 92%, 93%, 94%, 95%, 96%, 97%, 98% or 99% identical to the sequence as set forth in SEQ ID NO: 168 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 168 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 167.

As provided herein the biological sample used may be collected in a clinically acceptable manner, e.g. in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a tissue, for example a glandular tissue, e.g. the sample may be derived from the prostate of a subject. Additionally, cells may be purified from obtained body tissues and fluids if necessary, and then used as the biological sample. In some realizations, the sample may be a tissue sample, a fluid sample, a blood sample, a saliva sample, a sample including circulating tumour cells, extracellular vesicles, a sample containing prostate secreted exosomes, or cell lines or cancer cell line. In one particular realization, biopsy or resections samples may be obtained and/or used. Such samples may include cells or cell lysates.

Therefore, in an embodiment the biological sample obtained from a subject is a biopsy. In a further preferred embodiment, the method includes providing or obtaining a biopsy. In a preferred embodiment the biopsy is a prostate biopsy, for example a tissue or a fluid from the prostate, or a prostate cancer biopsy.

It is also conceivable that the content of a biological sample is submitted to an enrichment step. For instance, a sample may be contacted with ligands specific for the cell membrane or organelles of certain cell types, e.g. prostate cells, functionalized for example with magnetic particles. The material concentrated by the magnetic particles may subsequently be used for detection and analysis steps as described herein above or below.

Furthermore, cells, e.g. tumour cells, may be enriched via filtration processes of fluid or liquid samples, e.g. blood, urine, etc. Such filtration processes may also be combined with enrichment steps based on ligand specific interactions as described herein above.

It is preferred that a biological sample as provided herein is obtained from the subject before the start of the therapy. It is also preferred that said biological sample is a prostate sample or a prostate cancer sample.

Thus, in a preferred embodiment the method according to the invention comprises that the biological sample is obtained from the subject before the start of the therapy, preferably wherein the biological sample is a prostate sample or a prostate cancer sample. Alternatively, the method according to the invention comprises providing the biological sample obtained from the subject before the start of the therapy, preferably wherein the biological sample is a prostate sample or a prostate cancer sample.

It is contemplated herein that the outcome of a prostate cancer subject may be a favourable one or a non-favourable one. In one aspect of the invention a prediction of the outcome of a prostate cancer subject can result in the determination of a favourable risk or a non-favourable risk of a certain one or more outcomes. The outcomes may comprise prostate-cancer related death, loco-regional recurrence and/or distant recurrence. Preferably, prostate-cancer related death is prostate-cancer specific death.

Therefore, the present method provides for predicting a response of a subject to androgen deprivation therapy. The intent of androgen deprivation therapy is to eradicate malignant androgen-dependent cells, in the hope that sufficient tumor regression will permit complete resection of residual prostate cancer, improving pathologic outcome and survival. Therefore, for example the response may be measured as overall tumor size, tumor burden, number of tumor positive lymph nodes, probability of survival, overall survival time, cancer free survival time, overall chance of death, or cancer specific chance of death in response to androgen deprivation therapy. Therefore a response may be an increase or decreased predicted overall survival time when the androgen deprivation therapy is administered to the patient, compared to the situation where the androgen deprivation therapy is not administered, or the response may an increase or decreased predicted chance of cancer specific death when the androgen deprivation therapy is administered to the patient, compared to the situation where the androgen deprivation therapy is not administered. It is understood that other parameters as listed above could be used to determine the outcome of the androgen deprivation therapy. Therefore in an embodiment the prediction is based on at least one of: predicted reduced residual tumor burden; predicted reduced tumor size; and/or predicted reduced chance of tumor metastasis. In an embodiment prediction is a favourable or a non-favourable response to androgen deprivation therapy, wherein said favourable or non-favourable response is tumor size, tumor burden, number of tumor positive lymph nodes, probability of survival, overall survival, cancer free survival, overall death, or cancer specific death. In an embodiment said favourable outcome or non-favourable outcome is improved chance of survival in response to a treatment.

When used herein *tumor size* refers to the volume of a tumor at a predetermined time point after treatment. When used herein, *tumor burden* refers to the number of cancer cells, the size of the tumor or number of metastasis at a predetermined time point after treatment. When used herein *chance of tumor metastasis* refers to the chance that one or more metastasises will develop from the prostate cancer at a predetermined time point after treatment. When used herein number of positive lymph nodes refers the number of lymph nodes in the prostate cancer subject where at least one tumor cell can be identified. Preferably the cancer cell is a cancer cell derived from the prostate cancer. The positive lymph nodes may be local or peripheral. When used herein, probability of survival refers to the chance that the prostate cancer subject is still alive at a predetermined time point after treatment. When used herein the term *overall survival* refers to the (average) length of survival form the date of taking the biological sample from the subject. Cancer free survival refers to the (average) time the subject live without detectable cancer after the date of taking the biological sample from the subject. When used herein the term *overall death* refers to the (average) length until death from the date of taking the biological sample from the subject. When used herein the term *cancer specific death* refers to the (average) length until death caused as a result of the prostate cancer (or a metastasis thereof) from the date of taking the biological sample from the subject.

When used herein androgen deprivation therapy (ADT), also referred to as androgen suppression therapy, refers to an antihormone therapy. Androgen deprivation therapy aims to lower the levels of androgen hormones (such as testosterone) in the patient, as prostate cancer cells are usually dependent on androgen hormones for growth. ADT may comprise surgery based methods or drug based methods. For example orchiectomy (surgical removal of the testicles) may be used as ADT, as the testicles are main organ where androgens are produced. Alternatively a drug based method can be used, some non-limiting examples being chemical castration and antiandrogen treatment. Chemical castration may for example be achieved by agonists or antagonists of the gonadotropin releasing hormone (GnRH). GnRH induces Luteinizing Hormone production which in turn induces testosterone synthesis. GnRH agonists and antagonists used in androgen deprivation therapy include leuprorelin (leuprolide), goserelin, triptorelin, histrelin, buserelin, and degarelix. Antiandrogens treatment aims to reduce Androgen Receptor (AR) signaling induced androgen synthesis by blocking the AR signaling pathway, for example by sdisrupting the positive feedback loop created by testosterone, or alternatively target testosterone synthesis or AR nuclear translocation. Some exemplary examples of antiandrogens that may be used as ADT are cyproterone acetate, flutamide, nilutamide, bicalutamide, enzalutamide, abiraterone, abiraterone acetate, seviteronel, apalutamide, darolutamide, Leuprorelin and galeterone. Thus in an embodiment, the androgen deprivation therapy comprises treatment with an antiandrogen. In an embodiment the antiandrogen is selected from cyproterone acetate, flutamide, nilutamide, bicalutamide, enzalutamide, abiraterone, abiraterone acetate, seviteronel, apalutamide, darolutamide, Leuprorelin and galeterone, more preferably selected from enzalutamide, Leuprorelin, abiraterone, or apalutamide.

When used herein the term Neoadjuvant androgen deprivation therapy (NADT) refers to systemic therapy administered after the diagnosis of prostate cancer but before locoregional therapy such as radical prostatectomy (RP) or radiation. With NADT prior to RP, the intent is to eradicate malignant androgen-dependent cells, in the hope that sufficient tumor regression will permit complete resection of residual prostate cancer, improving pathologic outcome and survival. Therefore in an embodiment the androgen deprivation therapy is neoadjuvant androgen deprivation therapy.

A prediction provided by the method of the invention can provide for a prediction of the risk of a prostate cancer subject for a outcome of ADT. Moreover, the method of the invention can predict whether the subject having a prostate cancer has a low risk of a certain outcome or a high risk of a certain outcome. The outcomes prostate-cancer related death, loco-regional recurrence and/or distant recurrence, as used herein, comprise outcomes that are non-favourable to a subject. A further outcome is overall death. Overall death as used herein is an outcome that can be predicted by the method of the invention, but that is not directly associable with death of a subject due to the prostate cancer.

A prostate cancer subject having a high risk (i.e. above a certain threshold) to one or more of the outcomes prostate-cancer related death, loco-regional recurrence and/or distant recurrence, as predicted by the method of the invention, is contemplated to be associated with a non-favourable risk, preferably after surgery, for a respective outcome.

A prostate cancer subject having a low risk (i.e. below or equal to a certain threshold) to one or more of the outcomes prostate-cancer related death, loco-regional recurrence and/or distant recurrence, as predicted by the method of the invention, is contemplated to be associated with a favourable risk, preferably after surgery, for a respective outcome. A favourable risk may comprise a different follow-up strategy, for example a different recommended (follow-up) therapy, than a non-favourable risk for a subject. For example, a different follow-up strategy, for example a different recommended (follow-up) therapy, can be considered for a prostate cancer subject, preferably who has undergone surgery of the prostate (cancer), to improve survival chances for said prostate cancer subject.

It is preferred that a therapy comprises androgen deprivation therapy prior to locoregional therapy such as radical prostatectomy (RP) or radiation when a favourable outcome is predicted by the method defined herein. For example if it is predicted that the subject responds favourable to the ADT then ADT is administered prior to subsequent treatment. Favourable treatment may be implied by predicted linger survival, longer cancer-free survival etc as outlined above.

Thus, in a preferred embodiment the method according to the invention comprises that the biological sample, preferably the sample of a prostate of a subject or prostate cancer of a subject, is obtained prior to the start of therapy comprising surgery, radiotherapy, hormone therapy, cytotoxic chemotherapy and/or immunotherapy.

A non-favourable risk of an outcome is contemplated to impact the recommended therapy of said prostate cancer subject associated with the non-favourable risk. In a case wherein a non-favourable risk is predicted by the method of the invention it is contemplated that the recommended therapy comprises not providing androgen deprivation therapy to the subject.

In a preferred embodiment the method according to the invention comprises that a therapy is recommended based on the prediction, preferably the prediction on the outcome, wherein:

- if the prediction is non-favourable, the recommended therapy comprises not providing androgen deprivation therapy; and
- if the prediction is favourable, the recommended therapy comprises providing androgen deprivation therapy.

A favourable risk of an outcome is contemplated to impact the recommended therapy of said prostate cancer subject associated with the favourable risk. In a case wherein a favourable risk is predicted by the method of the invention it is contemplated that the recommended therapy comprises androgen deprivation therapy as broadly defined herein.

Thus, another preferred embodiment of the method according to the invention comprises that a therapy is recommended based on the prediction, wherein:

- if the prediction is favourable, the recommended therapy, comprises androgen deprivation therapy as defined herein.

In another aspect of the invention is provided a method in accordance with the invention comprising that secondary treatment is recommended for a certain patient, preferably for a patient having a low or a high risk of suffering an outcome selected from prostate-cancer specific death or overall death, wherein the recommendation is based on the prediction of the outcome of a prostate cancer patient, wherein:

- if the prediction is favourable no secondary treatment is recommended; and/or
- if the prediction is non-favourable secondary treatment is recommended.

It is provided herein that by means of the method in accordance with the invention the effectiveness of secondary treatment can be predicted post-surgery for patients having a low- or high-risk profile. Preferably, the secondary treatment comprises one or more, more preferably all, of chemotherapy, hormone therapy and radiation therapy.

The method as provided herein is based on the expression levels, e.g. expression profiles, of the PDE4D isoform 7 (PDE4D7) and/or at least three genes selected from PDE4D7 correlated genes selected from the group consisting of: ABCC5, CUX2, KIAA1549, PDE4D, RAP1GAP2, SLC39A11, TDRD1, and VWA2. The method may in addition be based on at least one gene selected from immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70. It is appreciated that the method may be performed on an input relating to the expression levels of the one or more genes, or that determining the expression levels may be part of the method.

It is further envisioned that the method is performed by a processor. Therefore, in an embodiment the invention relates to a computer implemented method of predicting an outcome of a prostate cancer subject.

It is preferred that the method for predicting a response of a prostate cancer subject to androgen deprivation therapy comprises determining the gene expression profile(s) of PDE4D7 and/or three or more, for example, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2. Optionally the gene expression profile may further comprise one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of one or more, for example, 1, 2, 3, 4, 5, 6, 7, 8, 9, 10, 11, 12, 13, 14, 15, 16 or all, T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70.

In a further embodiment the method for predicting a response of a prostate cancer subject to androgen deprivation therapy comprises determining the gene expression profile(s) of PDE4D7 and one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2.

In a further embodiment the gene expression profile may optionally comprise two or more, for example, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of two or more, for example, 2, 3, 4, 5, 6, 7, 8, 9, 10, 11, 12, 13, 14, 15, 16 or all, T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70.

In one preferred embodiment the method according to the invention the gene expression profile comprises:

- one or more immune defense response genes, preferably three or more, preferably, six or more, more preferably, nine or more, most preferably, all of the immune defense genes, and/or
- one or more T-Cell receptor signalling genes, preferably three or more, preferably, six or more, more preferably, nine or more, most preferably, all of the T-Cell receptor signalling genes, and
- the three or more PDE4D7 correlated genes, preferably four or more, preferably, six or more, most preferably, all of the PDE4D7 correlated genes.

Thus, it is preferred that the determining the first, second and/or third gene expression profile(s) according to the method of the invention comprises the determining or receiving the result of a determination of

- three or more, preferably six or more, more preferably, nine or more, most preferably, all of the genes selected from immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1,
- three or more, preferably six or more, more preferably, nine or more, most preferably, all of the genes selected from the group and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and
- three or more, preferably six or more, more preferably, nine or more, most preferably, all of the genes PDE4D7 correlated genes selected from the group consisting of: ABCC5, CUX2, KIAA1549, PDE4D, RAP1GAP2, SLC39A11, TDRD1, and VWA2.

In a further embodiment the method according to the invention comprises that the determining the prediction of the outcome is based on one or more immune defense response gene, one or more T-Cell receptor signalling gene in addition to PDE4D7 and/or three or more, for example three, four, five, six, seven or all, PDE4D7 correlated gene. In a further embodiment the method according to the invention comprises that the determining the prediction of the outcome is based on two or more immune defense response genes, two or more T-Cell receptor signalling genes in addition to PDE4D7 and/or three or more, for example three, four, five, six, seven or all, PDE4D7 correlated genes. In a further embodiment the method according to the invention comprises that the determining the prediction of the outcome is based on three or more immune defense response genes, three or more T-Cell receptor signalling genesin addition to PDE4D7 and/or three or more, for example three, four, five, six, seven or all, PDE4D7 correlated genes.

As a first reference example, the gene expression profile as embodied herein can further comprise at least one, at least two, at least three immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1. In one non-limiting example, the gene expression profile as embodied herein comprises the expression profiles of the genes DDX58, DHX9 and IFI16.

As a further reference example, the gene expression profile as embodied herein can further comprise at least one, at least two, at least three T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70. In one non-limiting example, the gene expression profile as embodied herein comprises the expression profiles of the genes PRKACA, PRKACB and PTPRC.

As a further reference example, the gene expression profile as embodied herein can comprise the PDE4D7 expression level and at least one, at least two, at least three PDE4D7 correlated genes selected from the group consisting of: ABCC5, CUX2, KIAA1549, PDE4D, RAP1GAP2, SLC39A11, TDRD1, and VWA2. In one non-limiting example, the gene expression profile as embodied herein comprises the expression profiles of the genes KIAA1549, PDE4D and RAP1GAP2.

As a further reference example, the gene expression profile as embodied herein can comprise at least three, at least four, at least five PDE4D7 correlated genes selected from the group consisting of: ABCC5, CUX2, KIAA1549, PDE4D, RAP1GAP2, SLC39A11, TDRD1, and VWA2. In one non-limiting example, the gene expression profile as embodied herein comprises the expression profiles of the genes CUX2, SLC39A11 and TDRD1.

Cox proportional-hazards regression allows analysing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g. death or disease recurrence) is called the hazard. Next to the information on whether or not the tested endpoint was reached by e.g. subject in a patient cohort (e.g. patient did die or not) also the time to the endpoint is considered in the regression analysis. The hazard is modelled as: $H(t) = Ho(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while $H(t)$ is the hazard at any time t. The hazard ratio (HR) (or the risk to reach the event) is represented by $Ln[H(t)/ Ho(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

Therefore in one preferred embodiment the method according to the invention comprises that the determining of the prediction of the outcome comprises combining the combination of the gene expression profile with a regression function that has been derived from a population of prostate cancer subjects. Therefore in an embodiment the method of the invention comprises that the prediction of the outcome comprises combining the three or more gene expression levels with a regression function that has been derived from a population of prostate cancer subjects.

In another preferred embodiment the method according to the invention comprises that the determining of the prediction of the outcome comprises:

- combining the first gene expression profiles for two or more, for example, 2, 3, 4, 5, 6, 7, 8, 9, 10, 11, 12, 13 or all, of the immune defense response genes with a regression function that has been derived from a population of prostate cancer subjects, and/or
- combining the second gene expression profiles for two or more, for example, 2, 3, 4, 5, 6, 7, 8, 9, 10, 11, 12, 13, 14, 15, 16 or all, of the T-Cell receptor signalling genes with a regression function that has been derived from a population of prostate cancer subjects, and/or
- combining the third gene expression profiles for two or more, for example, 2, 3, 4, 5, 6, 7 or all, of the PDE4D7 correlated genes with a regression function that has been derived from a population of prostate cancer subjects.

In one particular realization, the prediction of the outcome is determined as follows:

$$\text{PDE4D7\_CORR\_model:} \qquad\qquad\qquad (1)$$

$$(w_1 \cdot \text{ABCC5}) + (w_2 \cdot \text{CUX2}) + (w_3 \cdot \text{KIAA1549}) + [\ldots] + (w_8 \cdot \text{VWA2})$$

where $w_1$ to $w_8$ are weights and ABCC5, CUX2, KIAA1549, PDE4D, RAP1GAP2, SLC39A11, TDRD1, and VWA2 are the expression levels of genes.

In one particular realization, the prediction of the outcome is complemented with an additional model as follows:

$$\text{IDR\_14\_model:} \qquad\qquad\qquad (2)$$

$$(w_9 \cdot \text{AIM2}) + (w_{10} \cdot \text{APOBEC3A}) + (w_{11} \cdot \text{CIAO1}) + [\ldots] + (w_{22} \cdot \text{ZBP1})$$

where $w_9$ to $w_{22}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of genes.

In another particular realization, the prediction of the outcome is complemented with an additional model as follows:

$$\text{TCR\_17\_model:} \qquad\qquad\qquad (3)$$

$$(w_{23} \cdot \text{CD2}) + (w_{24} \cdot \text{CD247}) + (w_{25} \cdot \text{CD28}) + [\ldots] + (w_{39} \cdot \text{ZAP70})$$

where $w_{23}$ to $w_{39}$ are weights and CD2, CD247, CD28, CD3E, CD3G, CD4, CSK, EZR, FYN, LAT, LCK, PAG1, PDE4D, PRKACA, PRKACB, PTPRC, and ZAP70 are the expression levels of genes.

In another particular realization, the prediction of the outcome is determined as follows:

$$\text{PCAI\_model} \qquad\qquad\qquad (4)$$

$$(w_{40} \cdot \text{PDE4D7\_CORR}) + (w_{41} \cdot \text{PDE4D7})$$

In another realization, the prediction of the outcome is determined as follows:

PCAI_model                                                                      (5)

$$(w_1 \cdot ABCC5) + (w_2 \cdot CUX2) + (w_3 \cdot KIAA1549) + [\ldots] + (w_8 \cdot VWA2) + (w_{41} \cdot PDE4D7),$$

wherein $w_1$ to $w_8$ are weights and ABCC5, CUX2, KIAA1549, PDE4D, RAP1GAP2, SLC39A11, TDRD1, and VWA2 are the expression levels of genes.

The prediction of outcome may also be classified or categorized into one of at least two risk groups, based on the value of the prediction of the outcome. For example, there may be two risk groups, or three risk groups, or four risk groups, or more than four predefined risk groups.

Each risk group covers a respective range of (non-overlapping) values of the prediction of the outcome. For example, a risk group may indicate a probability of occurrence of a specific clinical event from 0 to <0.1 or from 0.1 to <0.25 or from 0.25 to <0.5 or from 0.5 to 1.0 or the like.

In a preferred embodiment the method according to the invention comprises that the determining of the prediction of the outcome is based on one or more clinical parameters obtained from the subject. As mentioned above, various measures based on one or more clinical parameters have been investigated. By predicating the prediction of the outcome upon such clinical parameter(s), it can be possible to further improve the prediction.

In an embodiment the determining of the prediction of the therapy response comprises combining the gene expression levels for PDE4D7 and/or three or more PDE4D7 correlated genes with a regression function that has been derived from a population of prostate cancer subjects.

It is further preferred that the gene expression profile for the PDE4D7 expression level and/or three or more PDE4D7 correlated genes and the one or more clinical parameters obtained from the subject are combined with a regression function that had been derived from a population of prostate cancer subjects.

It is also preferred that the one or more clinical parameters are combined with the gene expression profile with a regression function that is derived from a prostate cancer subject population in other to provide for a method for predicting a response of a prostate cancer subject to androgen deprivation therapy.

As such, in a preferred embodiment of the method according to the invention that the determining of the outcome comprises combining one or more of:

(i) the PDE4D7 expression level;
(ii) the expression levels of three or more PDE4D7 correlated genes;
(iii) the combination of the first gene expression profiles, second gene expression profiles, the third gene expression profiles, and the one or more clinical parameters obtained from the subject with a regression function that has been derived from a population of prostate cancer subjects.

In another particular realization, the prediction of the outcome is determined as follows:

PCAI_Clinical Model                                                             (6)

$$(w_{43} \cdot PDE4D7\_CORR) + (w_{44} \cdot LN\_positive)$$

where $w_{43}$ and $w_{44}$ are weights, PCAI_model is the above described regression model based on the expression profiles of one or more IDR genes, one or more TCR signalling genes and/or one or more PDE4D7 correlated genes, and LN_positive represents the number of tumour positive lymph nodes. Multi-variate Cox regression was used to create the clinical model combining the PCAI with clinical data (number of lymph node positive).

An example of a suitable clinical parameter, as has been used and exemplified herein, is "LN_positive" representing the number of tumour positive lymph nodes after post-surgical pathology. A LN positive prostate tumour is a tumour of the prostate wherein tumour cells have spread, i.e. metastasized, to nearby lymph nodes. It is contemplated that LN positive tumours are a harbinger for subclinical metastasis of the cancer.

It is understood to one skilled in the art that corresponding models can be formed for example for three or more PDE4D7 correlated genes, in which case an exemplare model would look like:

PDE4D7_CORR_EX1-3genes                                                          (7)

$$(w_3 \cdot KIAA1549) + (w_5 \cdot RAP1GAP2) + (w_7 \cdot TDRD1).$$

In a similar way components may be added to the model (e.g. one or more immune defense genes, or one or more T-Cell Receptor signalling genes). It is understood that when removing or adding components to the model, the weights are preferably re-calibrated for the amended model. The skilled person is aware that the model may be calibrated for example using a ground truth dataset comprising expression data obtained prior to ADT treatment in prostate cancer patients, wherein the dataset contains expression data from responders and non-responders to ADT treatment.

The method according to the invention may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

In a preferred embodiment the invention therefore also provides for a computer program product comprising instructions which, when the program is executed by a computer, cause the computer to carry out a method comprising:

- receiving

   the result of a determination of a gene expression profile
   comprising: the gene expression level of PDE4D7, and/or
   three or more gene expression levels, wherein the three or more gene expression levels are selected from:

   - PDE4D7 correlated genes selected from the group consisting of: ABCC5, CUX2, KIAA1549, PDE4D, RAP1GAP2, SLC39A11, TDRD1, and VWA2,

   said gene expression profile being determined in a biological sample obtained from the subject,

- determining the prediction of the outcome based on the gene expression profile,
- wherein said prediction is a favourable or a non-favourable response to androgen deprivation therapy. optionally, the method further provides the prediction of the outcome to a medical caregiver or the subject. In an embodiment the method further comprises receiving gene expression levels for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1. In an embodiment the method further comprises receiving gene expression levels for each of one or more, for example, 1, 2, 3, 4, 5, 6, 7, 8, 9, 10, 11, 12, 13, 14, 15, 16 or all, T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70. In an embodiment the method comprises receiving gene expression levels for each of three or more, for example, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the steps described herein can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

It is preferred herein that the computer program product according to the invention can be implemented on an apparatus for predicting a response of a prostate cancer subject to androgen deprivation therapy, wherein the apparatus comprises an input adapted to receive data indicative of a gene expression profile comprising the PDE4D7 expression level and/or three or more gene expression levels, wherein the three or more gene expression levels are selected from PDE4D7 correlated genes, and wherein said apparatus further comprises a processor adapted to determine the prediction of the response based on said gene expression profiles, and

- optionally, a providing unit adapted to provide the prediction or provide a therapy recommendation based on the selection to a medical caregiver or to the subject.

In a further aspect of the present invention is provided for an apparatus for predicting a response of a prostate cancer subject to androgen deprivation therapy, comprising:

- an input adapted to receive data indicative of a gene expression profile comprising the gene expression level of PDE4D7 and/or three or more gene expression levels, wherein the three or more gene expression levels are selected from:

  - each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2;

  said gene expression profiles being determined in a biological sample obtained from the subject,
- a processor adapted to determine the prediction of the outcome based on the gene expression profile, wherein said prediction is a favourable or a non-favourable response to androgen deprivation therapy, and
- optionally, a providing unit adapted to provide the prediction or a therapy recommendation based on the prediction to a medical caregiver or the subject.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

In a preferred embodiment the invention therefore also provides for a diagnostic kit, comprising at least one polymerase chain reaction primer, and optionally at least one probes, for determining the gene expression profile(s) in a biological sample obtained from a prostate cancer subject and/or in a sample.

It is preferred that said diagnostic kit as provided herein comprises at least one of: a polymerase chain reaction primer or probes, for determining a gene expression profile in a biological sample obtained from a prostate cancer subject and/or in a sample, the gene expression profile comprising:

the gene expression level of PDE4D7, and/or
three or more expression levels, wherein the three or more gene expression levels are selected from:

- PDE4D7 correlated genes selected from the group consisting of: ABCC5, CUX2, KIAA1549, PDE4D, RAP1GAP2, SLC39A11, TDRD1, and VWA2.

In another preferred embodiment the invention provides for the use of a diagnostic kit as broadly embodied herein in a method of predicting a response of a prostate cancer subject to androgen deprivation therapy, preferably for use in the method for predicting a response of a prostate cancer subject to androgen deprivation therapy as broadly embodied herein.

In an embodiment the invention relates to the use of a diagnostic kit, the kit comprising:

- at least one of: a polymerase chain reaction primer or probes, for determining a gene expression profile in a biological sample obtained from a prostate cancer subject and/or in a sample, the gene expression profile comprising:

  the gene expression level of PDE4D7, and/or
  three or more expression levels, wherein the three or more gene expression levels are selected from:

  - PDE4D7 correlated genes selected from the group consisting of: ABCC5, CUX2, KIAA1549, PDE4D, RAP1GAP2, SLC39A11, TDRD1, and VWA2,

  the use comprising predicting a response of a prostate cancer subject to androgen deprivation therapy, wherein the outcome is a favourable or non-favourable response to androgen deprivation therapy. In an embodiment the use comprises using the kit in the method as defined in a method for predicting a response of a prostate cancer subject to androgen deprivation therapy as broadly defined herein.

In another preferred embodiment the invention provides for a method, comprising:

- receiving a biological sample obtained from a prostate cancer subject,
- using the diagnostic kit, as broadly embodied herein, to determine the gene expression profile, the gene expression profile comprising:

the gene expression level of PDE4D7, and/or

three or more expression levels, wherein the three or more gene expression levels are selected from:

- PDE4D7 correlated genes selected from the group consisting of: ABCC5, CUX2, KIAA1549, PDE4D, RAP1GAP2, SLC39A11, TDRD1, and VWA2, in a biological sample obtained from a prostate cancer subject and/or in a sample.

All references cited herein, including journal articles or abstracts, published or corresponding patent applications, patents, or any other references, are entirely incorporated by reference herein, including all data, tables, figures, and text presented in the cited references. Additionally, the entire contents of the references cited within the references cited herein are also entirely incorporated by references.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

EXAMPLES

Generation of the different models based on Immune response defense genes, T-Cell receptor signalling genes or PDE4D7 correlated genes or combinations thereof have been described in:

Immune response defense genes WO 2021/175746
T-Cell receptor signalling genes WO 2021/175986
PDE4D7 correlated genes WO 2022/043120
Combination WO 2022/043299

These application further provide experimental evidence that predictions can be made based on part of the gene signature, such as 3, 4, 5, 6, 7 or 8 randomly selected genes from the Immune response defense genes, -Cell receptor signalling genes, or PDE4D7 correlated genes, or 3, 4, 5, 6, 7 or 8 randomly selected genes from a combination of these signatures.

Example 1: Nascent Prostate Cancer Heterogeneity Drives Evolution and Resistance to Intense Hormonal Therapy

*Background*

Patients diagnosed with high risk localized prostate cancer have variable outcomes following surgery. Trials of intense neoadjuvant androgen deprivation therapy (NADT) have shown lower rates of recurrence among patients with minimal residual disease after treatment. The molecular features that distinguish exceptional responders from poor responders are not known.

*Objective*

To identify genomic and histologic features associated with treatment resistance at baseline.

*Design, setting, and participants*

Targeted biopsies were obtained from 37 men with intermediate- to high-risk prostate cancer before receiving 6 months of ADT plus enzalutamide. Biopsy tissues were used for RNA sequencing. The publicly available dataset has been described in: Wilkinson et al. Nascent Prostate Cancer Heterogeneity Drives Evolution and Resistance to Intense Hormonal Therapy. Eur Urol 80 (2021), 746-757, hereby incorporated by reference in its entirety.

*Outcome measurements and statistical analysis*

Assessment of the relationship of molecular features of the pre-treatment biopsy tissue samples (n=35) with final pathologic response using a cut-point of 0.05 cm3 for residual cancer burden to compare exceptional responders to incomplete and non-responders.

*Overview of the study*

RNAseq based gene expression matrix of the study with n=35 eligible samples. The gene expression values for the genes of interest were used as input for data analysis. Calculation of the Prostate Cancer AI ImmunoScore based on a regression model previously developed on independent data (see e.g. WO 2022/043299). Testing of the association between the PCAI ImmunoScore and the response to 6 months neoadjuvant NADT. A schematic overview is depicted in Figure 1. The PCAI Immunoscore uses the PDE4D7 correlated genes and model described in WO 2022/043120.

*Results*

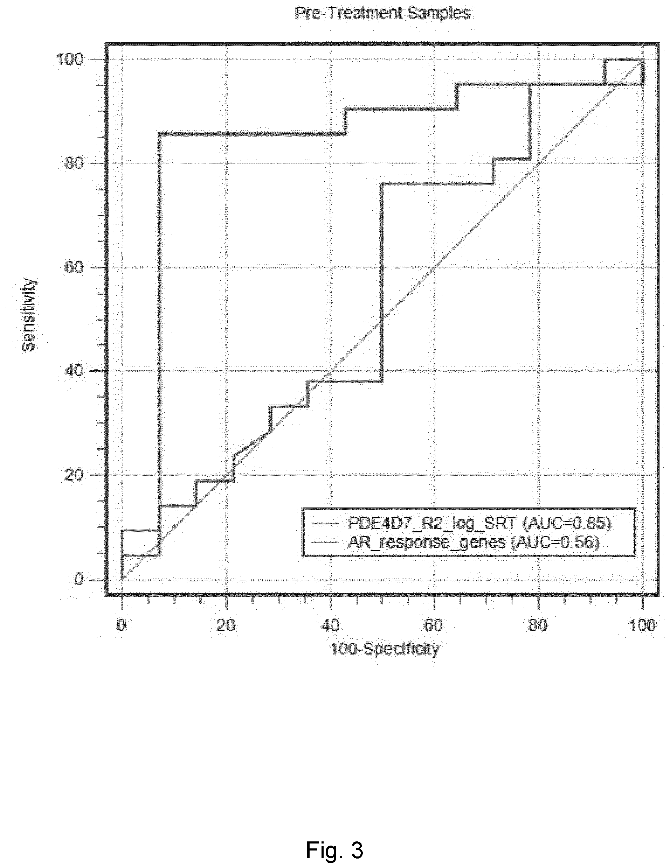

The results are displayed in Figure 3. The Prostate Cancer AI ImmunoScore accurately predicts exceptional treatment response to NADT (AUC=0.85) compared to a selection of genes that are regulated by the androgen receptor (AUC=0.56).

Example 2: Molecular features of exceptional response to NADT in high-risk localized prostate cancer

*Background*

High-risk localized prostate cancer is associated with a substantial risk of recurrence and disease mortality. Recent clinical trials have shown that intensifying anti-androgen therapies administered before prostatectomy can induce pathologic complete responses or minimal residual disease, called exceptional response, although the molecular determinants of these clinical outcomes are largely unknown.

*Objective*

To identify genomic and histologic features associated with treatment resistance at baseline.

*Design, setting, and participants*

Here, we perform whole-exome and transcriptome sequencing on pre-treatment multi-regional tumor biopsies from exceptional responders (ERs) and non-responders (NRs, pathologic T3 or lymph node-positive disease) to intensive neoadjuvant anti-androgen therapies (including apalutamide). The publicly available dataset has been described in Tewari et al. Molecular features of exceptional response to NADT in high-risk localized prostate cancer. Cell Reports 36 (2021), 109665, hereby incorporated by reference in its entirety.

*Outcome measurements and statistical analysis*

Assessment of the relationship of molecular features of the pre-treatment biopsy tissue samples (n=43) collected of n=24 patients with final pathologic response using a cut-point of <5 mm tumor tissue defined as exceptional responders compared to non-responders.

*Overview of the study*

RNAseq based gene expression matrix of the study with n=43 eligible samples. The gene expression values for the genes of interest were used as input for data analysis. Calculation of the Prostate Cancer AI ImmunoScore based on a regression model previously developed on independent data. Testing of the association between the PCAI ImmunoScore and the response to 6 months neoadjuvant NADT. A schematic overview is depicted in Figure 2. The PCAI Immunoscore uses the PDE4D7 correlated genes and model described in WO 2022/043120.

*Results*

The results are displayed in Figure 4. The Prostate Cancer AI ImmunoScore accurately predicts exceptional treatment response to NADT (AUC=0.92) compared to a selection of genes that are regulated by the androgen receptor (AUC=0.75).

**The attached Sequence Listing, entitled 2022PF00781 SEQ LIST.xml, is incorporated herein by reference, in its entirety.**

## Claims

1. A method of predicting a response of a prostate cancer subject to androgen deprivation therapy, comprising:

   - determining or receiving the result of a determination of a gene expression profile comprising: the gene expression level of PDE4D7, and/or

     three or more gene expression levels, wherein the three or more gene expression levels are selected from:

       - PDE4D7 correlated genes selected from the group consisting of: ABCC5, CUX2, KIAA1549, PDE4D, RAP1GAP2, SLC39A11, TDRD1, and VWA2,

     said gene expression profile being determined in a biological sample obtained from the subject,

   - determining the prediction of the outcome based on the gene expression profile,
   - wherein said prediction is a favourable or a non-favourable response to androgen deprivation therapy,
   - optionally, providing the prediction of the outcome to a medical caregiver or the subject.

2. Method according to claim 1, wherein the gene expression profile further comprises one or more gene expression level selected from:

   - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
   - T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70.

3. Method according to claim 1 or 2, wherein the androgen deprivation therapy is neoadjuvant androgen deprivation therapy.

4. Method according to any one of the preceding claims, wherein the androgen deprivation therapy comprises treatment with an antiandrogen,
   preferably wherein said antiandrogen is selected from cyproterone acetate, flutamide, nilutamide, bicalutamide, enzalutamide, abiraterone, abiraterone acetate, seviteronel, apalutamide, darolutamide, Leuprorelin and galeterone.

5. The method according to any one of the preceding claims, wherein the prediction is based on at least one of:

   - predicted reduced residual tumor burden;
   - predicted reduced tumor size;
   - predicted reduced chance of tumor metastasis.

6. The method according to any one of the preceding claims, wherein said favourable or non-favourable response is tumor size, tumor burden, number of tumor positive lymph nodes, probability of survival, overall survival, cancer free survival, overall death, or cancer specific death.

7. The method according to any one of the preceding claims, wherein said favourable outcome or non-favourable outcome is improved chance of survival in response to a treatment.

8. The method according to any one of the preceding claims, wherein the three or more genes comprise one or more immune defence response gene, one or more T-Cell receptor signalling gene and three or more PDE4D7 correlated genes.

9. The method according to claim 2 or claim 8, wherein:

- the one or more immune defense response genes comprise three or more, preferably, six or more, more preferably, nine or more, most preferably, all of the immune defense genes, and/or
- the one or more T-Cell receptor signalling genes comprise three or more, preferably, six or more, more preferably, nine or more, most preferably, all of the T-Cell receptor signalling genes, and/or
- the three or more PDE4D7 correlated genes comprise four or more, preferably, six or more, most preferably, all of the PDE4D7 correlated genes.

10. The method according to any one of the previous claims, wherein the determining of the prediction of the outcome comprises combining the three or more gene expression levels with a regression function that has been derived from a population of prostate cancer subjects.

11. The method according to any one of the preceding claims, wherein the determining of the prediction of the outcome is further based on one or more clinical parameters obtained from the subject.

12. The method according to any one of the preceding claims, wherein the determining of the outcome comprises combining the gene expression profile and one or more clinical parameters obtained from the subject with a regression function that has been derived from a population of prostate cancer subjects.

13. The method according to any one of the preceding claims, wherein the biological sample is obtained from the subject before the start of the therapy, preferably wherein the biological sample is a prostate sample or a prostate cancer sample.

14. The method according to any one of the preceding claims, wherein a therapy is recommended based on the prediction.

15. Use of a diagnostic kit, the kit comprising:

- at least one of: a polymerase chain reaction primer or probes, for determining a gene expression profile in a biological sample obtained from a prostate cancer subject and/or in a sample, the gene expression profile comprising:

the gene expression level of PDE4D7, and/or
three or more expression levels, wherein the three or more gene expression levels are selected from:

- PDE4D7 correlated genes selected from the group consisting of: ABCC5, CUX2, KIAA1549, PDE4D, RAP1GAP2, SLC39A11, TDRD1, and VWA2,

the use comprising predicting a response of a prostate cancer subject to androgen deprivation therapy, wherein the outcome is a favourable or non-favourable response to androgen deprivation therapy, preferably wherein the use comprises using the kit in the method as defined in any one of claims 1 to 14.

**Cohort #1 - #35 Patients**

- Intermediate to high-risk prostate cancer patients with localized disease
- ISUP Gleason 2-5 (ca 80% ISUP 4-5)
- Patients treated for 6 months with anti-androgens in a neo-adjuvant setting
- Biopsies collected from prostate before treatment start
- At the end of the hormonal treatment the prostate was resected by RP
- Tissue punches collected from resected prostate tissue
- Pre- and post-treatment tissues were subjected to RNAseq expression analysis
- Baseline tumor burden (BTB) available
- Residual cancer burden (RCB) available
- Patients separated into
  - exceptional responder (<=0.05 cm3 residual disease) intermediate (<=0.5 cm3 residual disease)
  - non-responders (>0.5 cm3 residual disease)

Testing of the Prostate Cancer AI (PCAI) ImmunoScore genomics signature

Fig. 1

**Cohort #2 - #24 Patients**

- High-risk prostate cancer patients with localized disease
- ISUP Gleason 2/3-5 (ca 75% ISUP 4-5)
- Patients treated for 6 months with anti-androgens in a neo-adjuvant setting (apalutamide, enzalutamide, Lupron, abiraterone)
- Multiple biopsies collected from prostate before treatment start (#43)
- At the end of the hormonal treatment the prostate was resected by RP
- Pre-treatment tissues were subjected to RNAseq expression analysis
- Patients separated into
  - exceptional responders (<5 mm tumor tissue),
  - non-responders

Testing of the Prostate Cancer AI (PCAI) ImmunoScore genomics signature

Fig. 2

Fig. 3

Fig. 4

Europäisches
Patentamt

European
Patent Office

Office européen
des brevets

# EUROPEAN SEARCH REPORT

**Application Number**

EP 22 20 2119

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/043299 A1 (KONINKLIJKE PHILIPS NV [NL]) 3 March 2022 (2022-03-03) | 1,2,8-15 | INV. C12Q1/6886 |
| Y | * abstract; claims 9-10 *<br>* page 37, line 33; claim 8 *<br>* page 39; claims 13-14 * | 4-7 | |
| X | PECHLIVANIS MAREE ET AL: "Biomarkers of Response to Neoadjuvant Androgen Deprivation in Localised Prostate Cancer", CANCERS, vol. 14, no. 1, 29 December 2021 (2021-12-29), page 166, XP093034031, DOI: 10.3390/cancers14010166 | 1-15 | |
| Y | * abstract; table 1 * | 1-15 | |
| X | TEWARI ALOK K. ET AL: "Molecular features of exceptional response to neoadjuvant anti-androgen therapy in high-risk localized prostate cancer", CELL REPORTS, vol. 36, no. 10, 1 September 2021 (2021-09-01), pages 109665-109665, XP093034033, US ISSN: 2211-1247, DOI: 10.1016/j.celrep.2021.109665 | 1-15 | |
| Y | * abstract; figure 3 *<br>* page 5, column 2 *<br>* Additional data: "Patient Cohort"; page e2 * | 1-15 | |

-----

-/--

**TECHNICAL FIELDS SEARCHED (IPC)**

C12Q

The present search report has been drawn up for all claims

| Place of search | Date of completion of the search | Examiner |
|---|---|---|
| The Hague | 23 March 2023 | Aguilera, Miguel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 22 20 2119

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WILKINSON SCOTT ET AL: "Nascent Prostate Cancer Heterogeneity Drives Evolution and Resistance to Intense Hormonal Therapy", EUROPEAN UROLOGY, ELSEVIER, AMSTERDAM, NL, vol. 80, no. 6, 27 March 2021 (2021-03-27), pages 746-757, XP086860889, ISSN: 0302-2838, DOI: 10.1016/J.EURURO.2021.03.009 [retrieved on 2021-03-27] | 1-15 | |
| Y | * abstract; figure 1 *<br>* sections 2.1, 3.1 * | 1-15 | |

TECHNICAL FIELDS SEARCHED (IPC)

The present search report has been drawn up for all claims

| Place of search | Date of completion of the search | Examiner |
|---|---|---|
| The Hague | 23 March 2023 | Aguilera, Miguel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 2119

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2023

| Patent document cited in search report | | Publication date | Patent family member(s) | | Publication date |
|---|---|---|---|---|---|
| WO 2022043299 | A1 | 03-03-2022 | EP | 3960877 A1 | 02-03-2022 |
| | | | WO | 2022043299 A1 | 03-03-2022 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2022043120 A
- WO 2021175746 A
- WO 2021175986 A
- WO 2022043299 A

### Non-patent literature cited in the description

- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- **MANTOVANI et al.** *Nature,* 2008, vol. 454 (7203), 436-44
- **GIRALDO et al.** *Br J Cancer.,* 2019, vol. 120 (1), 45-53
- **GIRALDO BR.** *J Cancer.,* 2019, vol. 120 (1), 45-53
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384
- **GASSER S. et al.** Sensing of dangerous DNA. *Mechanisms of Aging and Development,* 2017, vol. 165, 33-46
- **MOSENDEN R. ; TASKEN K.** Cyclic AMP-mediated immune regulation - Overview of mechanisms of action in T-cells. *Cell Signal,* 2011, vol. 23 (6), 1009-1016
- **TASKEN K. ; RUPPELT A.** Negative regulation of T-cell receptor activation by the cAMP-PKA-Csk signalling pathway in T-cell lipid rafts. *Front Biosci,* 2006, vol. 11, 2929-2939
- Immunity Leashed - Mechanisms of Regulation in the Human Immune System. **TORHEIM E.A.** Thesis for the degree of Philosophiae Doctor (PhD). The Biotechnology Centre of Ola, University of Oslo, 2009
- **TASKEN et al.** *Front in Bioscience,* 2006, vol. 11, 2929-2939
- **MOSENDEN et al.** *Cell Signalling,* 2011, vol. 23, 1009-1016
- **ABRAHAMSEN H. et al.** TCR- and CD28-mediated recruitment of phosphodiesterase 4 to lipid rafts potentiates TCR signaling. *J Immunol,* 2004, vol. 173, 4847-4848
- **WILKINSON et al.** Nascent Prostate Cancer Heterogeneity Drives Evolution and Resistance to Intense Hormonal Therapy. *Eur Urol,* 2021, vol. 80, 746-757
- **TEWARI et al.** Molecular features of exceptional response to NADT in high-risk localized prostate cancer. *Cell Reports,* 2021, vol. 36, 109665